# EUROPEAN PATENT SPECIFICATION

(11) **EP 3 464 617 B1**
(45) Date of publication and mention of the grant of the patent: **07.04.2021**
(21) Application number: 17727831.4
(22) Date of filing: 24.05.2017
(51) Int. Cl.: C12Q 1/68

(54) **COMPOSITIONS AND METHODS FOR DETECTION OF TRICHOMONAS VAGINALIS**
ZUSAMMENSETZUNGEN UND VERFAHREN ZUR DETEKTION VON TRICHOMONAS VAGINALIS
COMPOSITIONS ET MÉTHODES POUR LA DÉTECTION DE TRICHOMONAS VAGINALIS

(30) Priority: 27.05.2016 US 201662342600 P; 27.05.2016 US 201662342519 P
(43) Date of publication of application: 10.04.2019
(73) Proprietor: Roche Diagnostics GmbH, 68305 Mannheim (DE); F. Hoffmann-La Roche AG, 4070 Basel (CH)
(72) Inventor: HARRIS, Jody, Lafayette, California 94549 (US); LU, Shi Da Y., San Jose, California 95131 (US); MANGIPUDI, Kalyani, Pleasanton, California 94566 (US); SUN, Jingtao, San Ramon, California 94582 (US)
(74) Representative: Schwarz, Ralf
(86) International application number: PCT/EP2017/062509
(87) International publication number: WO 2017/202894

(56) References cited:
- WO-A2-2012/075317
- DWIVEDI S P ET AL: "18S ribosomal DNA based PCR diagnostic assay for Trichomonas vaginalis infection in symptomatic and asymptomatic women in India", ASIAN PACIFIC JOURNAL OF TROPICAL DISEASE 2012 ELSEVIER NLD, vol. 2, no. 2, April 2012 (2012-04), pages 133-138, XP002772260, ISSN: 2222-1808
- MAYTA H ET AL: "18S ribosomal DNA-based PCR for diagnosis of Trichomonas vaginalis", JOURNAL OF CLINICAL MICROBIOLOGY 2000 US, vol. 38, no. 7, 2000, pages 2683-2687, XP002772262, ISSN: 0095-1137
- HOBBS MARCIA M ET AL: "Modern diagnosis of Trichomonas vaginalis infection", SEXUALLY TRANSMITTED INFECTIONS, BMJ PUBLISHING GROUP, LONDON, GB , vol. 89, no. 6, Sp. Iss. SI 30 April 2013 (2013-04-30), pages 434-438, XP002740077, ISSN: 1368-4973, DOI: 10.1136/SEXTRANS-2013-051057 Retrieved from the Internet: URL:http://www.ncbi.nlm.nih.gov/pmc/articl es/PMC3787709/pdf/nihms-517699.pdf
- LILIA TORRES-MACHORRO ANA ET AL: "Comparative analyses among the Trichomonas vaginalis, Trichomonas tenax, and Tritrichomonas foetus 5S ribosomal RNA genes", CURRENT GENETICS, vol. 55, no. 2, April 2009 (2009-04), pages 199-210, XP019708943,

## Description

### FIELD OF THE INVENTION

The present disclosure relates to the field of molecular diagnostics, and more particularly to detection of *Trichomonas vaginalis.*

### BACKGROUND OF THE INVENTION

*Trichomonas vaginalis* (TV) is a flagellated protozoan parasite that causes trichomoniasis, the most prevalent nonviral sexually transmitted infection in the United States, affecting an estimated 3.7 million persons nationwide. The prevalence differs among black and non-Hispanic white women with 13% compared to 1.8% affected, respectively. *T. vaginalis* infection has been reported to affect >11% of women aged ≥40 years and prevalence rates have been reported as high as 26% in symptomatic women and 6.5% in asymptomatic women tested at STD clinics. *T. vaginalis* is also known to cause urethritis in men who have sex with women (MSW). Most infections go unnoticed, with 70% of men and 85% women experiencing only minor symptoms and if untreated may last for months or years. Asymptomatic spread of infection does occur and remains a problem. Infections in women include vaginitis, cervicitis and urethritis. Symptomatic women usually complain of vaginal discharge, vulvovaginal soreness, and/or irritation. Dysuria is also common. Complications can include premature labor, low-birth-weight offspring, premature rupture of membranes, and post-abortion or post-hysterectomy infection. An association with pelvic inflammatory disease (PID), tubal infertility, and cervical cancer with previous episodes of trichomoniasis has been reported. Symptoms in men may include urethritis, epididymitis, or prostatitis.

*T. vaginalis* infection is associated with two-to three-fold increased risk for HIV acquisition, preterm birth, and other adverse pregnancy outcomes among pregnant women. Among women with HIV infection, *T. vaginalis* infection is associated with increased risk for pelvic inflammatory disease (PID). Routine screening of asymptomatic women with HIV infection for *T. vaginalis* is recommended because of the adverse events associated with asymptomatic trichomoniasis and HIV infection. Diagnostic testing for *T. vaginalis* should be performed in women seeking care for vaginal discharge. Screening might be considered for persons receiving care in high-prevalence settings (e.g. STD clinics and correctional facilities) and for asymptomatic persons at high risk for infection (e.g. persons with multiple sex partners, illicit drug use, or a history of STD).

Before molecular methods became available, culture was considered the gold standard method for diagnosing *T. vaginalis* infection but the sensitivity of culture has been estimated to range from 38% to 82% when compared to molecular methods. For culture in women, vaginal secretions are the preferred over urine since urine culture has been shown to be less sensitive. In men, culture specimens require a urethral swab, urine sediment, and/or semen. Culture of multiple specimens from men used to inoculate a single culture may improve sensitivity. The microscopic examination of wet preparations of genital secretions is probably the most common method for *T. vaginalis* diagnosis because of convenience and relatively low cost but is only 35% to 80% sensitive compared with culture. Moreover, the sensitivity of the wet-mount method is highly dependent on the experience of the microscopist as well as the time of specimen transport to the laboratory where sensitivity declines by up to 20% within 1 hour after collection. Thus there is a need in the art for a quick and reliable method to specifically detect TV in a sensitive manner.

### SUMMARY OF THE INVENTION

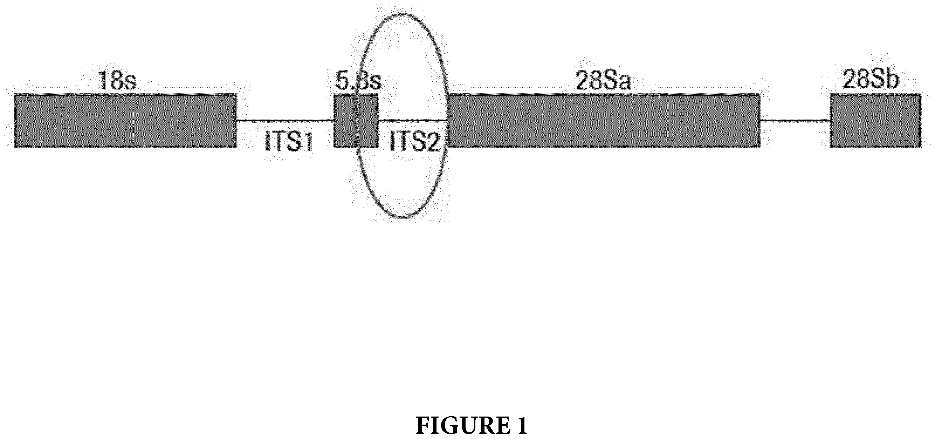

Certain embodiments in the present disclosure relate to methods for the rapid detection of the presence or absence of TV in a biological or non-biological sample, for example, multiplex detection of TV by real-time polymerase chain reaction in a single test tube. Embodiments include methods of detection of TV comprising performing at least one cycling step, which may include an amplifying step and a hybridizing step. Furthermore, embodiments include primers, probes, and kits that are designed for the detection of TV in a single tube. The detection methods are designed to target specific genes in the *T. vaginalis* genome with a potential to discriminate against the nearest neighbors *Trichomonas tenax* and *Pentatrichomonas hominis.* A method for detecting TV in a sample is provided, including performing an amplifying step including contacting the sample with a set of primers designed to target a specific TV gene to produce an amplification product if TV is present in the sample; performing a hybridizing step including contacting the amplification product with one or more detectable probes to the target TV gene; and detecting the presence or absence of the amplified product, wherein the presence of the amplified product is indicative of the presence of TV in the sample and wherein the absence of the amplified product is indicative of the absence of TV in the sample; wherein the target TV gene is the 5.8 s ribosomal RNA (5.8s) gene. Figure 1 shows the location of the 5.8s gene, the inter-transcribed sequence 2 (ITS2) and neighboring genes in the TV genome.

In one aspect a method of detecting *Trichomonas vaginalis* (TV) in a sample is provided, the method comprising performing an amplifying step comprising contacting the sample with a set of target TV gene primers to produce an amplification product if a target TV gene nucleic acid is present in the sample; performing a hybridizing step comprising contacting the amplification product with one or more detectable target TV gene probes; and detecting the presence or absence of the amplification product, wherein the presence of the amplification product is indicative of the presence of TV in the sample and wherein the absence of the amplification product is indicative of the absence of TV in the sample; wherein the set of target TV gene primers comprise a first primer comprising or consisting of a first oligonucleotide sequence selected from the group consisting of SEQ ID NOs: 1-9, or a complement thereof, and a second primer comprising or consisting of a second oligonucleotide sequence selected from the group consisting of SEQ ID NOs: 10-13, or a complement thereof; and wherein the one or more detectable target TV gene probes comprises or consists of a third oligonucleotide sequence selected from the group consisting of SEQ ID NOs: 14-18, or the complement thereof.

In one embodiment, the primer set for amplification of the 5.8s gene target includes a first primer comprising or consisting of a first oligonucleotide sequence selected from the group consisting of SEQ ID NOs: 1, 2, 3, 4, 5, 6, 7, 8, and 9, or a complement thereof, and a second primer comprising or consisting of a second oligonucleotide sequence selected from the group consisting of SEQ ID NOs: 10, 11, 12, and 13, or a complement thereof, and the detectable probe for detection of the 5.8s gene amplification product includes or consists of the nucleic acid sequences of SEQ ID NOs: 14, 15, 16, 17, and 18, or a complement thereof. In certain embodiments, the primer set for amplification of the 5.8s gene target includes a first primer comprising or consisting of a first oligonucleotide sequence selected from the group consisting of SEQ ID NOs: 3, 6, 7, and 9, or a complement thereof, and a second primer comprising or consisting of a second oligonucleotide sequence selected from the group consisting of SEQ ID NO: 12, or a complement thereof, and the detectable probe for detection of the 5.8s gene amplification product includes or consists of the nucleic acid sequences of SEQ ID NOs: 16, 17, and 18, or a complement thereof.

In some embodiments the hybridizing step comprises contacting the amplification product with the detectable target TV gene probe that is labeled with a donor fluorescent moiety and a corresponding acceptor moiety; and the detecting step comprises detecting the presence or absence of fluorescence resonance energy transfer (FRET) between the donor fluorescent moiety and the acceptor moiety of the probe, wherein the presence or absence of fluorescence is indicative of the presence or absence of TV in the sample. In some embodiments the amplifying and the hybridizing steps are repeated. Herein, the number of repetitions depends, e.g., on the nature of the sample. If the sample is a complex mixture of nucleic acids, more amplifying and hybridizing steps will be required to amplify the target sequence sufficient for detection. In some embodiments, the amplifying and the hybridizing steps are repeated at least about 20 times, but may be repeated as many as at least 25, 30, 40, 50, 60, or even 100 times. Further, detecting the presence or absence of the amplification product may be performed during or after each amplifying and hybridizing step, during or after every other amplifying and hybridizing step, during or after particular amplifying and hybridizing steps or during or after particular amplifying and hybridizing steps, in which - if present - sufficient amplification product for detection is expected. In some embodiments, the amplifying step employs a polymerase enzyme having 5' to 3' nuclease activity. In some embodiments, the donor fluorescent moiety and the corresponding acceptor moiety are within no more than 8-20 nucleotides of each other on the probe. In some embodiments, the acceptor moiety is a quencher.

In some embodiments the oligonucleotides comprise or consist of a sequence of nucleotides selected from SEQ ID NOs: 1-18, or a complement thereof have 100 or fewer nucleotides, 50 or fewer nucleotides, 40 or fewer nucleotides or 30 or fewer nucleotides. In some embodiments, the first and second target TV gene primers and detectable target TV gene probe have 40 or fewer nucleotides (e.g. 35 or fewer nucleotides, 30 or fewer nucleotides, etc.). In some embodiments, the oligonucleotides comprise at least one modified nucleotide, e.g., to alter nucleic acid hybridization stability relative to unmodified nucleotides. Optionally, the oligonucleotides comprise at least one label and/or at least one quencher moiety. In some embodiments, the oligonucleotides include at least one conservatively modified variation. "Conservatively modified variations" or, simply, "conservative variations" of a particular nucleic acid sequence refers to those nucleic acids, which encode identical or essentially identical amino acid sequences, or, where the nucleic acid does not encode an amino acid sequence, to essentially identical sequences. One of skill will recognize that individual substitutions, deletions or additions which alter, add or delete a single amino acid or a small percentage of amino acids (typically less than 5%, more typically less than 4%, 2% or 1%) in an encoded sequence are "conservatively modified variations" where the alterations result in the deletion of an amino acid, addition of an amino acid, or substitution of an amino acid with a chemically similar amino acid. In some embodiments, at least one of the first and second target TV gene primers and detectable target TV gene probe comprises at least one modified nucleotide.

In some embodiments, amplification (the amplifying step) can employ a polymerase enzyme having 5' to 3' nuclease activity. Thus, the donor fluorescent moiety and the acceptor moiety, e.g., a quencher, may be within no more than 5 to 20 nucleotides (e.g., 8 or 10) of each other along the length of the probe. In another aspect, the detectable probe includes a nucleic acid sequence that permits secondary structure formation. Such secondary structure formation generally results in spatial proximity between the first and second fluorescent moiety.

According to this method, the second fluorescent moiety on the probe can be a quencher. The present disclosure provides for methods of detecting the presence or absence of TV in a biological sample from an individual. Such methods generally include performing at least one cycling step, which includes an amplifying step and a dye-binding step. Typically, the amplifying step includes contacting the sample with a plurality of pairs of primers designed to target a specific TV gene to produce one or more target TV gene amplification products if the target TV gene nucleic acid molecule is present in the sample, and the dye-binding step includes contacting the target TV gene amplification product with a double-stranded DNA binding dye. Such methods also include detecting the presence or absence of binding of the double-stranded DNA binding dye into the amplification product, wherein the presence of binding is indicative of the presence of TV in the sample, and wherein the absence of binding is indicative of the absence of TV in the sample. A representative double-stranded DNA binding dye is ethidium bromide. In addition, such methods also can include determining the melting temperature between the target TV gene amplification product and the double-stranded DNA binding dye, wherein the melting temperature confirms the presence or absence of TV. The target TV gene may include but is not limited to the 5.8s gene, the 18s gene, the PMS1 gene, the Mlhla gene, and the CRN gene.

In another aspect, a kit for detecting one or more nucleic acids of TV is provided. The kit can include one or more sets of primers specific for amplification of the target TV gene; and one or more detectable probes specific for detection of the target TV gene amplification products. The target TV gene may include but is not limited to the 5.8s gene, the 18s gene, the PMS1 gene, the Mlhla gene, and the CRN gene. In particular, the oligonucleotide primers and probes disclosed above in connection with the method according to the invention are suitable to being included in a kit according to the invention. Herein, a kit for detecting a nucleic acid of *Trichomonas vaginalis* (TV) is provided comprising a first primer comprising a first oligonucleotide sequence selected from the group consisting of SEQ ID NOs: 1-9, or a complement thereof; a second primer comprising a second oligonucleotide sequence selected from the group consisting of SEQ ID NOs: 10-13, or a complement thereof; and a fluorescently detectably labeled probe comprising a third oligonucleotide sequence selected from the group consisting of SEQ ID NOs: 14-18, or a complement thereof, the detectably labeled probe configured to hybridize to an amplicon generated by the first primer and the second primer. In one aspect, the kit can include probes already labeled with donor and corresponding acceptor moiety, e.g., another fluorescent moiety or a dark quencher, or can include fluorophoric moieties for labeling the probes. The kit can also include at least one of nucleoside triphosphates, nucleic acid polymerase, and buffers necessary for the function of the nucleic acid polymerase. The kit can also include a package insert and instructions for using the primers, probes, and fluorophoric moieties to detect the presence or absence of TV in a sample. In some embodiments, the third detectably labeled oligonucleotide sequence comprises a donor fluorescent moiety and a corresponding acceptor moiety. In some embodiments, the acceptor moiety is a quencher. In some embodiments, at least one of the first, second, and third oligonucleotides comprises at least one modified nucleotide. In some embodiments, the first, second, and third oligonucleotides have 40 or fewer nucleotides.

In another aspect, compositions are provided comprising a set of oligonucleotide primers for amplifying a target TV gene as disclosed above. In some embodiments, the set of target TV gene primers comprises a first primer comprising or consisting of a first oligonucleotide sequence selected from the group consisting of SEQ ID NOs: 1-9, or a complement thereof, and a second primer comprising or consisting of a second oligonucleotide sequence selected from the group consisting of SEQ ID NOs: 10-13, or a complement thereof. In certain embodiments the composition further comprises one or more detectable target TV gene probes comprises or consists of a third oligonucleotide sequence selected from the group consisting of SEQ ID NOs: 14-18, or the complement thereof.

In another aspect, the methods of detecting TV in a biological sample from an individual are conducted together with methods to detect *Mycoplasma genitalium* (MG) from the same biological sample due to the asymptomatic nature of individuals infected with TV and/or MG. Herein, the method for detecting MG and TV in a sample in addition to the steps for amplification and detection of TV provided above further includes performing an amplifying step including contacting the sample with a set of primers designed to target a specific MG gene to produce an amplification product if MG is present in the sample; performing a hybridizing step including contacting the amplification product with one or more detectable probes to the target MG gene; and detecting the presence or absence of the amplified product, wherein the presence of the amplified product is indicative of the presence of MG in the sample and wherein the absence of the amplified product is indicative of the absence of MG in the sample; wherein the target MG gene is selected from the group consisting of the 23s ribosomal RNA (23s) gene, the conserved region A of the mgpB gene within the MgPa adhesion operon (mgpB), and the variable EF region of the mgpB partial repeats (MgPar). In one embodiment, the methods of detecting TV and MG in the biological sample are performed in the same reaction mixture as a multiplex assay. In one embodiment the set of target MG gene primers comprise a first primer comprising a first oligonucleotide sequence selected from the group consisting of SEQ ID NOs: 106-112, 134-140, and 152-161 or a complement thereof, and a second primer comprising a second oligonucleotide sequence selected from the group consisting of SEQ ID NOs: 113-122, 141-146, and 162-171, or a complement thereof; and wherein the one or more detectable target MG gene probes comprises a third oligonucleotide sequence selected from the group consisting of SEQ ID NOs: 123-133, 147-151, and 172-194, or the complement thereof. In one embodiment, the primer set for amplification of the MG 23s gene target includes a first primer comprising or consisting of a first oligonucleotide sequence selected from the group consisting of SEQ ID NOs: 106, 107, 108, 109, 110, 111, and 112, or a complement thereof, and a second primer comprising or consisting of a second oligonucleotide sequence selected from the group consisting of SEQ ID NOs: 113, 114, 115, 116, 117, 118, 119, 120, 121, and 122, or a complement thereof, and the detectable probe for detection of the MG 23s gene amplification product includes or consists of the nucleic acid sequences of SEQ ID NOs: 123, 124, 125, 126, 127, 128, 129, 130, 131, 132, and 133, or a complement thereof. In certain embodiments, the primer set for amplification of the MG 23s gene target includes a first primer comprising or consisting of a first oligonucleotide sequence selected from the group consisting of SEQ ID NOs: 108 and 109, or a complement thereof, and a second primer comprising or consisting of a second oligonucleotide sequence selected from the group consisting of SEQ ID NOs: 113 and 114, or a complement thereof, and the detectable probe for detection of the MG 23s gene amplification product includes or consists of the nucleic acid sequences of SEQ ID NO: 129, or a complement thereof.

In another embodiment, the primer set for amplification of the mgpB gene target includes a first primer comprising or consisting of a first oligonucleotide sequence selected from the group consisting of SEQ ID NOs: 134, 135, 136, 137,138, 139, and 140, or a complement thereof, and a second primer comprising or consisting of a second oligonucleotide sequence selected from the group consisting of SEQ ID NOs: 141, 142, 143, 144, 145, and 146, or a complement thereof, and the detectable probe for detection of the mgpB gene amplification product includes or consists of the nucleic acid sequences of SEQ ID NOs: 147, 148, 149, 150, and 151, or a complement thereof. In certain embodiments, the primer set for amplification of the mgpB gene target includes a first primer comprising or consisting of a first oligonucleotide sequence selected from the group consisting of SEQ ID NOs: 139 and 140, or a complement thereof, and a second primer comprising or consisting of a second oligonucleotide sequence selected from the group consisting of SEQ ID NOs: 145 and 146, or a complement thereof, and the detectable probe for detection of the mgpB gene amplification product includes or consists of the nucleic acid sequences of SEQ ID NOs: 150 and 151, or a complement thereof.

In another embodiment, the primer set for amplification of the MgPar gene target includes a first primer comprising or consisting of a first oligonucleotide sequence selected from the group consisting of SEQ ID NOs: 152, 153, 154, 155, 156, 157, 158, 159, 160, and 161, or a complement thereof, and a second primer comprising or consisting of a second oligonucleotide sequence selected from the group consisting of SEQ ID NOs: 162, 163, 164, 165, 166, 167, 168, 169, 170, and 171, or a complement thereof, and the detectable probe for detection of the MgPar gene amplification product includes or consists of the nucleic acid sequences of SEQ ID NOs: 172, 173, 174, 175, 176, 177, 178, 179, 180, 181, 182, 183, 184, 185, 186, 187, 188, 189, 190, 191, 192, 193, and 194, or a complement thereof. In certain embodiments, the primer set for amplification of the MgPar gene target includes a first primer comprising or consisting of a first oligonucleotide sequence selected from the group consisting of SEQ ID NOs: 153 and 161, or a complement thereof, and a second primer comprising or consisting of a second oligonucleotide sequence selected from the group consisting of SEQ ID NOs: 169, 170 and 171, or a complement thereof, and the detectable probe for detection of the MgPar gene amplification product includes or consists of the nucleic acid sequences of SEQ ID NOs: 185 and 194, or a complement thereof. In some embodiments, the hybridizing step further comprises contacting the amplification product with the detectable target MG gene probe that is labeled with a donor fluorescent moiety and a corresponding acceptor moiety; and the detecting step comprises detecting the presence or absence of fluorescence resonance energy transfer (FRET) between the donor fluorescent moiety and the acceptor moiety of the probe, wherein the presence or absence of fluorescence is indicative of the presence or absence of MG in the sample. In some embodiments the amplifying and the hybridizing steps are repeated. Herein, the number of repetitions depends, e.g., on the nature of the sample. If the sample is a complex mixture of nucleic acids, more amplifying and hybridizing steps will be required to amplify the target sequence sufficient for detection. In some embodiments, the amplifying and the hybridizing steps are repeated at least about 20 times, but may be repeated as many as at least 25, 30, 40, 50, 60, or even 100 times. Further, detecting the presence or absence of the amplification product may be performed during or after each amplifying and hybridizing step, during or after every other amplifying and hybridizing step, during or after particular amplifying and hybridizing steps or during or after particular amplifying and hybridizing steps, in which - if present - sufficient amplification product for detection is expected. In some embodiments, the amplifying step employs a polymerase enzyme having 5' to 3' nuclease activity. In some embodiments, the donor fluorescent moiety and the corresponding acceptor moiety are within no more than 8-20 nucleotides of each other on the probe. In some embodiments, the acceptor moiety is a quencher. In some embodiments the oligonucleotides comprise or consist of a sequence of nucleotides selected from SEQ ID NOs: 106-194, or a complement thereof have 100 or fewer nucleotides, 50 or fewer nucleotides, 40 or fewer nucleotides or 30 or fewer nucleotides. In some embodiments, the first and second target MG gene primers and detectable target MG gene probe have 40 or fewer nucleotides (e.g. 35 or fewer nucleotides, 30 or fewer nucleotides, etc.). In some embodiments, the oligonucleotides comprise at least one modified nucleotide, e.g., to alter nucleic acid hybridization stability relative to unmodified nucleotides. Optionally, the oligonucleotides comprise at least one label and/or at least one quencher moiety. In some embodiments, the oligonucleotides include at least one conservatively modified variation. "Conservatively modified variations" or, simply, "conservative variations" of a particular nucleic acid sequence refers to those nucleic acids, which encode identical or essentially identical amino acid sequences, or, where the nucleic acid does not encode an amino acid sequence, to essentially identical sequences. One of skill will recognize that individual substitutions, deletions or additions which alter, add or delete a single amino acid or a small percentage of amino acids (typically less than 5%, more typically less than 4%, 2% or 1%) in an encoded sequence are "conservatively modified variations" where the alterations result in the deletion of an amino acid, addition of an amino acid, or substitution of an amino acid with a chemically similar amino acid. In some embodiments, at least one of the first and second target MG gene primers and detectable target MG gene probe comprises at least one modified nucleotide.

In another aspect a kit designed for the combined detection of TV and MG in a single tube is provided. Herein, the kit can include one or more sets of primers specific for amplification of the target TV gene; and one or more detectable probes specific for detection of the target TV gene amplification products as provided above in combination with one or more sets of primers specific for amplification of the target MG gene; and one or more detectable probes specific for detection of the target MG gene amplification products. In one embodiment the kit comprises a first primer comprising a first oligonucleotide sequence selected from the group consisting of SEQ ID NOs: 1-9, or a complement thereof; a second primer comprising a second oligonucleotide sequence selected from the group consisting of SEQ ID NOs: 10-13, or a complement thereof; and a fluorescently detectably labeled probe comprising a third oligonucleotide sequence selected from the group consisting of SEQ ID NOs: 14-18, or a complement thereof, the detectably labeled probe configured to hybridize to an amplicon generated by the first primer and the second primer and further comprising a set of primers designed to target a specific MG gene to produce an amplification product of said MG gene and one or more detectable probes capable to hybridize to the MG amplification product. Herein, said specific MG gene is selected from the group consisting of the 23s ribosomal RNA (23s) gene, the conserved region A of the mgpB gene within the MgPa adhesion operon (mgpB), and the variable EF region of the mgpB partial repeats (MgPar). More specifically, the primers and probes for amplification and detection of the specific MG gene may be the MG primers and probes described above. In some embodiments the set of target MG gene primers comprise a first primer comprising a first oligonucleotide sequence selected from the group consisting of SEQ ID NOs: 106-112, 134-140, and 152-161 or a complement thereof, and a second primer comprising a second oligonucleotide sequence selected from the group consisting of SEQ ID NOs: 113-122, 141-146, and 162-171, or a complement thereof; and wherein the one or more detectable target MG gene probes comprises a third oligonucleotide sequence selected from the group consisting of SEQ ID NOs: 123-133, 147-151, and 172-194, or the complement thereof. In some embodiments, the kit includes probes already labeled with donor and corresponding acceptor moiety, e.g., another fluorescent moiety or a dark quencher, or can include fluorophoric moieties for labeling the probes. Herein, the labels for the probes for detecting TV and for detecting MG may be the same. In this embodiment the kit may be used for detecting either of TV or MG without differentiating which target nucleic acid is present. In another embodiment the label(s) for the probes for detecting TV differ from the label(s) for detecting MG allowing for the specific detection of either target nucleic acid and differentiating which target nucleic acid is present (either none of TV and MG, only one of either TV or MG, or both TV and MG). The kit may further comprise any of the components outlined for the TV kits above.

Unless otherwise defined, all technical and scientific terms used herein have the same meaning as commonly understood by one of ordinary skill in the art to which this invention belongs. Although methods and materials similar or equivalent to those described herein can be used in the practice or testing of the present subject matter, suitable methods and materials are described below. In addition, the materials, methods, and examples are illustrative only and not intended to be limiting. The details of one or more embodiments of the invention are set forth in the accompanying drawings and the description below. Other features, objects, and advantages of the invention will be apparent from the drawings and detailed description, and from the claims.

### BRIEF DESCRIPTION OF THE FIGURES

FIGURE 1 shows the location of the 5.8s ribosomal RNA gene, the inter-transcribed sequence 2 (ITS2) and neighboring ribosomal genes in the *T. vaginalis* genome.
FIGURE 2 shows PCR growth curves of a real-time PCR experiment in the presence of various concentrations of genomic *T. vaginalis* DNA template (present in 1000 [black], 100 [light grey] and 10 [grey] genomic equivalent concentrations per PCR reaction).
FIGURES 3 and 4 show PCR growth curves of a real-time PCR experiment with concentrations of genomic *T. vaginalis* DNA template present in 100 (1e2 FdT), 10 (1e1 FdT), 5 (5ge FdT) and 1 (lge FdT) genomic equivalent concentrations per PCR reaction (ge/PCR), in a co-amplification with internal control standard (GIC 10PFU/PCR) and *Mycoplasma genitalium* DNA template at 10 ge/PCR (MG 10ge/PCR).

### DETAILED DESCRIPTION OF THE INVENTION

Diagnosis of TV infection by nucleic acid amplification provides a method for rapidly and accurately detecting the protozoan infection. A real-time assay for detecting TV in a sample is described herein. Primers and probes for detecting TV are provided, as are articles of manufacture or kits containing such primers and probes. The increased sensitivity of real-time PCR for detection of TV compared to other methods, as well as the improved features of real-time PCR including sample containment and real-time detection of the amplified product, make feasible the implementation of this technology for routine diagnosis of TV infections in the clinical laboratory.

The present disclosure includes oligonucleotide primers and fluorescent labeled hydrolysis probes that hybridize to a specific gene locus of the TV genome in order to specifically identify TV using TaqMan^{®} amplification and detection technology. Target selection for TV required a comprehensive search of the public sequence database, as well as literature search for TV targets with a potential to discriminate against the nearest neighbors, *Trichomonas tenax* and *Pentatrichomonas hominis.* Multiple targets from the public sequence database were analyzed in the target selection process but many showed cross reactivity with *T. tenax* and P. *hominis.* Furthermore, sequences in the public database are complicated by "bulk" sequence data from multicopy targets.

As a result of the analysis, possible target TV genes include the 5.8s ribosomal RNA gene (GenBank accession number U86613), the 18s ribosomal RNA gene (GenBank accession number NW001533462), the DNA mismatch repair homolog, post-meiotic segregation increased-1 (PMS1) gene (GenBank accession number NW001581675), the MutL homolog 1a (Mlhla) gene (GenBank accession number XM001320341) and the coronin (CRN) gene (GenBank accession number XM001581132). In certain aspects, the target TV gene is the 5.8s ribosomal RNA gene as this gene is present in over 200 copies per genome and will hence benefit sensitivity of the assay. Further, the 5.8s ribosomal RNA gene is present in metronidazole resistant and metronidazole sensitive TV and hence allows for the combined detection of both TV types.

The disclosed methods may include performing at least one cycling step that includes amplifying one or more portions of the nucleic acid molecule gene target from a sample using one or more pairs of primers. "Primer(s)" as used herein refer to oligonucleotide primers that specifically anneal to the target gene in TV, and initiate DNA synthesis therefrom under appropriate conditions producing the respective amplification products. Each of the discussed primers anneals to a target within or adjacent to the respective target nucleic acid molecule such that at least a portion of each amplification product contains nucleic acid sequence corresponding to the target. The one or more amplification products are produced provided that one or more of the target TV gene nucleic acid is present in the sample, thus the presence of the one or more of target TV gene amplification products is indicative of the presence of TV in the sample. The amplification product should contain the nucleic acid sequences that are complementary to one or more detectable probes for target TV gene. "Probe(s)" as used herein refer to oligonucleotide probes that specifically anneal to nucleic acid sequence encoding the target TV gene. Each cycling step includes an amplification step, a hybridization step, and a detection step, in which the sample is contacted with the one or more detectable probes for detection of the presence or absence of TV in the sample.

As used herein, the term "amplifying" refers to the process of synthesizing nucleic acid molecules that are complementary to one or both strands of a template nucleic acid molecule. Amplifying a nucleic acid molecule typically includes denaturing the template nucleic acid, annealing primers to the template nucleic acid at a temperature that is below the melting temperatures of the primers, and enzymatically elongating from the primers to generate an amplification product. Amplification typically requires the presence of deoxyribonucleoside triphosphates, a DNA polymerase enzyme (e.g., Platinum® Taq) and an appropriate buffer and/or co-factors for optimal activity of the polymerase enzyme (e.g., MgCl₂ and/or KCl).

The term "primer" as used herein is known to those skilled in the art and refers to oligomeric compounds, primarily to oligonucleotides but also to modified oligonucleotides that are able to "prime" DNA synthesis by a template-dependent DNA polymerase, i.e., the 3'-end of the, e.g., oligonucleotide provides a free 3'-OH group whereto further "nucleotides" may be attached by a template-dependent DNA polymerase establishing 3' to 5' phosphodiester linkage whereby deoxynucleoside triphosphates are used and whereby pyrophosphate is released. Therefore, there is - except possibly for the intended function - no fundamental difference between a "primer", an "oligonucleotide", or a "probe".

The term "hybridizing" refers to the annealing of one or more probes to an amplification product. Hybridization conditions typically include a temperature that is below the melting temperature of the probes but that avoids non-specific hybridization of the probes.

The term "5' to 3' nuclease activity" refers to an activity of a nucleic acid polymerase, typically associated with the nucleic acid strand synthesis, whereby nucleotides are removed from the 5' end of nucleic acid strand.

The term "thermostable polymerase" refers to a polymerase enzyme that is heat stable, i.e., the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus, T. ruber, T. thermophilus, T. aquaticus, T. lacteus, T. rubens, Bacillus stearothermophilus,* and *Methanothermus fervidus.* Nonetheless, polymerases that are not thermostable also can be employed in PCR assays provided the enzyme is replenished.

The term "complement thereof" refers to nucleic acid that is both the same length as, and exactly complementary to, a given nucleic acid.

The term "extension" or "elongation" when used with respect to nucleic acids refers to when additional nucleotides (or other analogous molecules) are incorporated into the nucleic acids.

For example, a nucleic acid is optionally extended by a nucleotide incorporating biocatalyst, such as a polymerase that typically adds nucleotides at the 3' terminal end of a nucleic acid.

The terms "identical" or percent "identity" in the context of two or more nucleic acid sequences, refer to two or more sequences or subsequences that are the same or have a specified percentage of nucleotides that are the same, when compared and aligned for maximum correspondence, e.g., as measured using one of the sequence comparison algorithms available to persons of skill or by visual inspection. Exemplary algorithms that are suitable for determining percent sequence identity and sequence similarity are the BLAST programs, which are described in, e.g., Altschul et al. (1990) "Basic local alignment search tool" J. Mol. Biol. 215:403-410, Gish et al. (1993) "Identification of protein coding regions by database similarity search" Nature Genet. 3:266-272, Madden et al. (1996) "Applications of network BLAST server" Meth. Enzymol. 266:131-141, Altschul et al. (1997) "Gapped BLAST and PSI-BLAST: a new generation of protein database search programs" Nucleic Acids Res. 25:3389-3402, and Zhang et al. (1997) "PowerBLAST: A new network BLAST application for interactive or automated sequence analysis and annotation" Genome Res. 7:649-656.

A "modified nucleotide" in the context of an oligonucleotide refers to an alteration in which at least one nucleotide of the oligonucleotide sequence is replaced by a different nucleotide that provides a desired property to the oligonucleotide. Exemplary modified nucleotides that can be substituted in the oligonucleotides described herein include, e.g., a C5-methyl-dC, a C5-ethyl-dC, a C5-methyl-dU, a C5-ethyl-dU, a 2,6-diaminopurine, a C5-propynyl-dC, a C5-propynyl-dU, a C7-propynyl-dA, a C7-propynyl-dG, a C5-propargylamino-dC, a C5-propargylamino-dU, a C7-propargylamino-dA, a C7-propargylamino-dG, a 7-deaza-2-deoxyxanthosine, a pyrazolopyrimidine analog, a pseudo-dU, a nitro pyrrole, a nitro indole, 2'-0-methyl Ribo-U, 2'-0-methyl Ribo-C, an N4-ethyl-dC, an N6-methyl-dA, and the like. Many other modified nucleotides that can be substituted in the oligonucleotides are referred to herein or are otherwise known in the art. In certain embodiments, modified nucleotide substitutions modify melting temperatures (Tm) of the oligonucleotides relative to the melting temperatures of corresponding unmodified oligonucleotides. To further illustrate, certain modified nucleotide substitutions can reduce non-specific nucleic acid amplification (e.g., minimize primer dimer formation or the like), increase the yield of an intended target amplicon, and/or the like in some embodiments. Examples of these types of nucleic acid modifications are described in, e.g., U.S. Pat. No. 6,001,611.

### Detection of TV

The present disclosure provides methods to detect TV by amplifying, for example, a portion of the target TV gene nucleic acid sequence. Nucleic acid sequences of the 5.8s gene. Specifically, primers and probes to amplify and detect specific TV nucleic acid molecule targets are provided by the embodiments in the present disclosure.

For detection of TV, primers and probes to amplify the target TV gene are provided. Nucleic acids other than those exemplified herein can also be used to detect TV in a sample. For example, functional variants can be evaluated for specificity and/or sensitivity by those of skill in the art using routine methods. Representative functional variants can include, e.g., one or more deletions, insertions, and/or substitutions in the target TV gene nucleic acids disclosed herein.

More specifically, embodiments of the oligonucleotides each include a nucleic acid with a sequence selected from SEQ ID NOs: 1-9, 10-13, and 14-18.

**TABLE I: 5.8s Forward Primers**

| **Forward Primers** | | | |
|---|---|---|---|
| **Oligo Name** | **SEQ ID NO:** | **Sequence** | **Modifications** |
| KMTV5.8s110FBBC | 1 | CCAAGTCTCTAAGCAATGGATGT<t_BB_dC> | t-butylbenzyldC |
| KMTV5.8s117FBBC | 2 | AAGCAATGGATGTCTTGGCT<t_BB_dC> | t-butylbenzyldC |
| KMTV5.8s169FBBC | 3 | TGTTAAGTAACCGGAGTTGCAAA<t_BB_dC> | t-butylbenzyldC |
| KMTV170FBBC | 4 | TTAAGTAACCGGAGTTGCAAA<t_BB_dC> | t-butylbenzyldC |
| KMTV195FBBC | 5 | CAAATTGCGCTAAACTCGATCT<t_BB_dC> | t-butylbenzyldC |
| KMTV203FBBA | 6 | CTAAACTCGATCTCGGTCG<t_BB_dA> | t-butylbenzyldA |
| KMTV201FBBC | 7 | CGCTAAACTCGATCTCGGT<t_BB_dC> | t-butylbenzyldC |
| KMTV196FBBC | 8 | AAATTGCGCTAAACTCGATCT<t_BB_dC> | t-butylbenzyldC |
| KMTV194FBBC | 9 | GCAAATTGCGCTAAACTCGAT<t_BB_dC> | t-butylbenzyldC |

**TABLE II: 5.8s Reverse Primers**

| **Reverse Primers** | | | |
|---|---|---|---|
| **Oligo Name** | **SEQ ID NO:** | **Sequence** | **Modifications** |
| KMTV5.8s220RBBA | 10 | TCACACCCATGCTTCTCG<t_BB_dA> | t -butylbenzyldA |
| KMTV5.8s212RBBC | 11 | CATGCTTCTCGACCGAGAT<t_BB_dC> | t-butylbenzyldC |
| KMTV5.8s268RBBA | 12 | TGTTTGTCTTATATATTATTTACTTATTCGCT TAGA<t_BB_dA> | t-butylbenzyldA |
| KMTV270RBBA | 13 | TTTGTCTTATATATTATTTACTTATTCGCTTA GA<t_BB_dA> | t-butylbenzyldA |

**TABLE III: 5.8s Probes**

| **Probes** | | | |
|---|---|---|---|
| **Oligo Name** | **SEQ ID NO:** | **Sequence** | **Modifications** |
| KMTV5.8s167FQ6 | 14 | <F>AACATC<Q>ATGACAGGTTAATCTT TGAATGCAAATTG<P> | P=phosphate, F=th-FAM, Q=BHQ2 |
| KMTV5.8s153FQ6 | 15 | <F>TAACCG<Q>GAGTTGCAAACATCAT GACAGG<P> | P=phosphate, F=th-FAM, Q=BHQ2 |
| KMTV5.8s198FQ6 | 16 | <F>ATTGCG<Q>CTAAACTCGATCTCGG TCGA<P> | P=phosphate, F=th-FAM, Q=BHQ2 |
| KMTV5.8s204FQ6 | 17 | <F>CTAAAC<Q>TCGATCTCGGTCGAGA AGCATGG<P> | P=phosphate, F=th-FAM, Q=BHQ2 |
| KMTV5.8s220FQ6 | 18 | <F>TCGAGA<Q>AGCATGGGTGTGACAG TACTACATCT<P> | P=phosphate, F=th-FAM, Q=BHQ2 |

**TABLE IV: 18s Forward Primers**

| **Forward Primers** | | | |
|---|---|---|---|
| **Oligo Name** | **SEQ ID NO:** | **Sequence** | **Modifications** |
| 16S101BZ | 19 | CGTAGTTGGGATTGACGTTTGTAATC<t_BZ_dA> | t-benzyldA |
| 16S101BU | 20 | CGTAGTTGGGATTGACGTTTGTAATC<t_BB_dA> | t-butylbenzyldA |
| 16S103BZ | 21 | GGGAAACTTACCAGGACCAG<t_BZ_dA> | t-benzyldA |
| 16S103BU | 22 | GGGAAACTTACCAGGACCAG<t_BB_dA> | t-butylbenzyldA |
| 16S105BZ | 23 | GAAACTTACCAGGACCAGATGTTTTTT<t_BZ_dA> | t-benzyldA |
| 16S105BU | 24 | GAAACTTACCAGGACCAGATGTTTTTT<t_BB_dA> | t-butylbenzyldA |
| 16S107BZ | 25 | CTTGAAGGAATTGACGGAAGGGCAC<t_BZ_dA> | t-benzyldA |
| 16S107BU | 26 | CTTGAAGGAATTGACGGAAGGGCAC<t_BB_dA> | t-butylbenzyldA |
| 16S109BZ | 27 | GCCATTCGACTGAGTGACCTATC<t_BZ_dA> | t-benzyldA |
| 16S109BU | 28 | GCCATTCGACTGAGTGACCTATC<t_BB_dA> | t -butylbenzyldA |

**TABLE V: 18s Reverse Primers**

| **Reverse Primers** | | | |
|---|---|---|---|
| **Oligo Name** | **SEQ ID NO:** | **Sequence** | **Modifications** |
| 16S102BZ | 29 | GACTTCTCCTTCCTCTAGATAACGTG<t_BZ_dA> | t-benzyldA |
| 16S102BU | 30 | GACTTCTCCTTCCTCTAGATAACGTG<t_BB_dA> | t -butylbenzyldA |
| 16S104BZ | 31 | TTGCTACCCTCTTCCACCTGCTAA<t_BZ_dA> | t-benzyldA |
| 16S104BU | 32 | TTGCTACCCTCTTCCACCTGCTAA<t_BB_dA> | t-butylbenzyldA |
| 16S106BZ | 33 | GCTACCCTCTTCCACCTGCTAAAAT<t_BZ_dC> | t-benzyldC |
| 16S106BU | 34 | GCTACCCTCTTCCACCTGCTAAAAT<t_BB_dC> | t-butylbenzyldC |
| 16S108BZ | 35 | TGAATCAACGCTAGACAGGTCAA<t_BZ_dC> | t-benzyldC |
| 16S108BU | 36 | TGAATCAACGCTAGACAGGTCAA<t_BB_dC> | t-butylbenzyldC |
| 16S110BZ | 37 | AAAAGGCACCAATGGAACTCGTCATT<t_BZ_dA> | t-benzyldA |
| 16S110BU | 38 | AAAAGGCACCAATGGAACTGGTCATT<t_BB_dA> | t-butylbenzyldA |

**TABLE VI: 18s Probes**

| **Probes** | | | |
|---|---|---|---|
| **Oligo Name** | **SEQ ID NO:** | **Sequence** | **Modifications** |
| 16S141FQ6 | 39 | <F>AATCCC<Q>TTGTAAATGTGTGTCAACAA CGCA<P> | P=phosphate, F=th-FAM, Q=BHQ2 |
| 16S142FQ6 | 40 | <F>CCACCA<Q>AAAACAATATCCTGAAAGAC CCGAAG<P> | P=phosphate, F=th-FAM, Q=BHQ2 |
| 16S144FQ6 | 41 | <F>CCAAAA<Q>ACAATATCCTGAAAGACCCG AAGCCT<P> | P=phosphate, F=th-FAM, Q=BHQ2 |
| 16S146FQ6 | 42 | <F>ACCAAA<Q>AACAATATCCTGAAAGACCC GAAGCC<P> | P=phosphate, F=th-FAM, Q=BHQ2 |
| 16S148FQ6 | 43 | <F>CTGCTA<Q>CCCGTGGATATAGTCGCTAT CTCTC<P> | P=phosphate, F=th-FAM, Q=BHQ2 |
| 16S143FQ6 | 44 | <F>CTGAGA<Q>GATAGCGACTATATCCACGG GTAGC<P> | P=phosphate, F=th-FAM, Q=BHQ2 |

**TABLE VII: PMS1 Forward Primers**

| **Forward Primers** | | | |
|---|---|---|---|
| **Oligo Name** | **SEQ ID NO:** | **Sequence** | **Modifications** |
| PMS103BZ | 45 | CCGAGAGATGATTGAGAACGTATTTG<t_BZ_dA> | t-benzyldA |
| PMS103BU | 46 | CCGAGAGATGATTGAGAACGTATTTG<t_BB_dA> | t -butylbenzyldA |
| PMS107BZ | 47 | CACTCCGAGAGATGATTGAGAACGT<t_BZ_dA> | t-benzyldA |
| PMS107BU | 48 | CACTCCGAGAGATGATTGAGAACGT <t_BB_dA> | t-butylbenzyldA |

**TABLE VIII: PMS1 Reverse Primers**

| **Reverse Primers** | | | |
|---|---|---|---|
| **Oligo Name** | **SEQ ID NO:** | **Sequence** | **Modifications** |
| PMS104BZ | 49 | GCCACTTACATCTTTTCCAAATT<t_BZ_dC> | t-benzyldC |
| PMS104BU | 50 | GCCACTTACATCTTTTCCAAATT<t_BB_dC> | t-butylbenzyldC |
| PMS108BZ | 51 | GTGACACCTTCATCACAAATCATTGAA<t_BZ_dA> | t-benzyldA |
| PMS108BU | 52 | GTGACACCTTCATCACAAATCATTGAA<t_BB_dA> | t-butylbenzyldA |

**TABLE IX: PMS1 Probes**

| **Probes** | | | |
|---|---|---|---|
| **Oligo Name** | **SEQ ID NO:** | **Sequence** | **Modifications** |
| PMA144FQ6 | 53 | <F>CCACCA<Q>TTTCCAACTCGAATTGTCAA AAGT<P> | P=phosphate, F=th-FAM, Q=BHQ2 |
| PMS146FQ6 | 54 | <F>CCACCA<Q>TTTCCAACTCGAATTGTCAA AAGTGT<P> | P=phosphate, F=th-FAM, Q=BHQ2 |

**TABLE X: Mlhla Forward Primers**

| **Forward Primers** | | | |
|---|---|---|---|
| **Oligo Name** | **SEQ ID NO:** | **Sequence** | **Modifications** |
| MLH101BZ | 55 | CTCCTGTATCTATAAATGAAGAGA<t_BZ_dA> | t - benzyldA |
| MLH101BU | 56 | CTCCTGTATCTATAAATGAAGAGA<t_BB_dA> | t-butylbenzyldA |
| MLH103BZ | 57 | GATTTCTGATAATGGCTGTGGAATAA<t_BZ_dA> | t - benzyldA |
| MLH103BU | 58 | GATTTCTGATAATGGCTGTGGAATAA<t_BB_dA> | t-butylbenzyldA |
| MLH105BZ | 59 | GAATTATCTCCTGTATCTATAAATGA<t_BZ_dA> | t - benzyldA |
| MLH105BU | 60 | GAATTATCTCCTGTATCTATAAATGA<t_BB_dA> | t -butylbenzyldA |
| MLH107BZ | 61 | AGTAACAGCAAGTTCACTTTTGT<t_BZ_dC> | t-benzyldC |
| MLH107BU | 62 | AGTAACAGCAAGTTCACTTTTGT<t_BB_dC> | t-butylbenzyldC |
| MLH109BZ | 63 | CAGGTGATATCGCGAAGAACAC<t_BZ_dA> | t - benzyldA |
| MLH109BU | 64 | CAGGTGATATCGCGAAGAACAC<t_BB_dA> | t-butylbenzyldA |

**TABLE XI: Mlhla Reverse Primers**

| **Reverse Primers** | | | |
|---|---|---|---|
| **Oligo Name** | **SEQ ID NO:** | **Sequence** | **Modifications** |
| MLH102BZ | 65 | GGAATATTTGATTTTGGAATTTCAG<t_BZ_dA> | t - benzyldA |
| MLH102BU | 66 | GGAATATTTGATTTTGGAATTTCAG<t_BB_dA> | t-butylbenzyldA |
| MLH104BZ | 67 | CCAAATGAACTTTCTTCTGTTTTAG<t_BZ_dA> | t - benzyldA |
| MLH104BU | 68 | CCAAATGAACTTTCTTCTGTTTTAG<t_BB_dA> | t-butylbenzyldA |
| MLH106BZ | 69 | ATTTGATTTTGGAATTTCAGAGTTTT<t_BZ_dC> | t-benzyldC |
| MLH106BU | 70 | ATTTGATTTTGGAATTTCAGAGTTTT<t_BB_dC> | t-butylbenzyldC |
| MLH108BZ | 71 | CTGAAGACTTGGAATAGATGTACTG<t_BZ_dC> | t-benzyldC |
| MLH108BU | 72 | CTGAAGACTTGGAATAGATGTACTG<t_BB_dC> | t-butylbenzyldC |
| MLH110BZ | 73 | GGCATCCTTAATAAAACAAAAGCAA<t_BZ_dA> | t - benzyldA |
| MLH110BU | 74 | GGCATCCTTAATAAAACAAAAGCAA<t_BB_dA> | t-butylbenzyldA |

**TABLE XII: Mlh1a Probes**

| **Probes** | | | |
|---|---|---|---|
| **Oligo Name** | **SEQ ID NO:** | **Sequence** | **Modifications** |
| MLH141FQ6 | 75 | <F>AAAATC<Q>CAAGAAAAAGAGCAAGAA GAAATCCT<P> | P=phosphate, F=th-FAM, Q=BHQ2 |
| MLH142FQ6 | 76 | <F>CACCTC<Q>TGAATCCAAATGTAGTTAC GTTCCTT<P> | P=phosphate, F=th-FAM, Q=BHQ2 |
| MLH144FQ6 | 77 | <F>CTTGCT<Q>CTTTTCTTGGATTTTCTGT ATCTGA<P> | P=phosphate, F=th-FAM, Q=BHQ2 |
| MLH146FQ6 | 78 | <F>TTCAAA<Q>CCAATCAAACCAACAAAAG AATGAGC<P> | P=phosphate, F=th-FAM, Q=BHQ2 |
| MLH148FQ6 | 79 | <F>AAAACC<Q>GCTGATTCTTTGAGTTGTT TTTTGGC<P> | P=phosphate, F=th-FAM, Q=BHQ2 |

**TABLE XIII: CRN Forward Primers**

| **Forward Primers** | | | |
|---|---|---|---|
| **Oligo Name** | **SEQ ID NO:** | **Sequence** | **Modifications** |
| CRN101BZ | 80 | GCAATCTGGGATCTCAACAAGGAA<t_BZ_dA> | t-benzyldA |
| CRN101BU | 81 | GCAATCTGGGATCTCAACAAGGAA<t_BB_dA> | t-butylbenzyldA |
| CRN103BZ | 82 | TTTCATCGGACAGGGCAATC<t_BZ_dC> | t-benzyldC |
| CRN103BU | 83 | TTTCATCGGACAGGGCAATC<t_BB_dC> | t-butylbenzyldC |
| CRN105BZ | 84 | GAGGGACCACAAGAAGAAGTCGTTC<t_BZ_dA> | t-benzyldA |
| CRN105BU | 85 | GAGGGACCACAAGAAGAAGTCGTTC<t_BB_dA> | t-butylbenzyldA |
| CRN107BZ | 86 | GACGAGGGACCACAAGAAGAAGT<t_BZ_dC> | t-benzyldC |
| CRN107BU | 87 | GACGAGGGACCACAAGAAGAAGT<t_BB_dC> | t-butylbenzyldC |
| CRN109BZ | 88 | CAGAGATCATCCAGCCAGAT<t_BZ_dC> | t-benzyldC |
| CRN109BU | 89 | CAGAGATCATCCAGCCAGAT<t_BB_dC> | t -butylbenzyldC |

**TABLE XIV: CRN Reverse Primers**

| **Reverse Primers** | | | |
|---|---|---|---|
| **Oligo Name** | **SEQ ID NO:** | **Sequence** | **Modifications** |
| CRN102BZ | 90 | GGTTGTAATCTGGAAGGTCGAGA<t_BZ_dA> | t-benzyldA |
| CRN102BU | 91 | GGTTGTAATCTGGAAGGTCGAGA<t_BB_dA> | t-butylbenzyldA |
| CRN104BZ | 92 | AACGTCAGGAACATCCCAAAGG<t_BZ_dC> | t-benzyldC |
| CRN104BU | 93 | AACGTCAGGAACATCCCAAAGG<t_BB_dC> | t-butylbenzyldC |
| CRN106BZ | 94 | GCGAGTTGGCTTATCAAGGTTCATGA<t_BZ_dA> | t-benzyldA |
| CRN106BU | 95 | GCGAGTTGGCTTATCAAGGTTCATGA<t_BB_dA> | t-butylbenzyldA |
| CRN108BZ | 96 | GTTGATTGGATAGCGAGTTGG<t_BZ_dC> | t-benzyldC |
| CRN108BU | 97 | GTTGATTGGATAGCGAGTTGG<t_BB_dC> | t-butylbenzyldC |
| CRN110BZ | 98 | CTCGTCGACAACTTCCTCCT<t_BZ_dC> | t-benzyldC |
| CRN110BU | 99 | CTCGTCGACAACTTCCTCCT<t_BZ_dC> | t -butylbenzyldC |

**TABLE XV: CRN Probes**

| **Probes** | | | |
|---|---|---|---|
| **Oligo Name** | **SEQ ID NO:** | **Sequence** | **Modifications** |
| CRN141FQ6 | 100 | <F>CCAAGA<Q>CTCAACATTCAACTCATTAT CTAACG<P> | P=phosphate, F=th-FAM, Q=BHQ2 |
| CRN143FQ6 | 101 | <F>ACATCA<Q>CATACTCACCACATAATCCA AATCT<P> | P=phosphate, F=th-FAM, Q=BHQ2 |
| CRN142FQ6 | 102 | <F>ACAATG<Q>CCAACTGGAAGATAAGTAAA GTAGT<P> | P=phosphate, F=th-FAM, Q=BHQ2 |
| CRN144FQ6 | 103 | <F>ACAATG<Q>CCAACTGGAAGATAAGTAAA GTAGTT<P> | P=phosphate, F=th-FAM, Q=BHQ2 |
| CRN146FQ6 | 104 | <F>TTGGAA<Q>GATGGGATTTGTCAACTGTC AATCTG<P> | P=phosphate, F=th-FAM, Q=BHQ2 |
| CRN148FQ6 | 105 | <F>TTGGAA<Q>GATGGGATTTGTCAACTGTC AATCT<P> | P=phosphate, F=th-FAM, Q=BHQ2 |

In one embodiment, the above described sets of primers and probes are used in order to provide for detection of TV in a biological sample suspected of containing TV. The sets of primers and probes may comprise or consist of the primers and probes specific for the nucleic acid sequences of the 5.8s gene.The primer (and/or probe) may be chemically modified, i.e., a primer and/or probe may comprise a modified nucleotide or a non-nucleotide compound. A probe (or a primer) is then a modified oligonucleotide. "Modified nucleotides" (or "nucleotide analogs") differ from a natural "nucleotide" by some modification but still consist of a base or base-like compound, a pentofuranosyl sugar or a pentofuranosyl sugar-like compound, a phosphate portion or phosphate-like portion, or combinations thereof. For example, a "label" may be attached to the base portion of a "nucleotide" whereby a "modified nucleotide" is obtained. A natural base in a "nucleotide" may also be replaced by, e.g., a 7-deazapurine whereby a "modified nucleotide" is obtained as well. The terms "modified nucleotide" or "nucleotide analog" are used interchangeably in the present application. A "modified nucleoside" (or "nucleoside analog") differs from a natural nucleoside by some modification in the manner as outlined above for a "modified nucleotide" (or a "nucleotide analog"). Oligonucleotides including modified oligonucleotides and oligonucleotide analogs that amplify a nucleic acid molecule encoding the target TV gene, e.g. the 5.8s gene, can be designed using, for example, a computer program such as OLIGO (Molecular Biology Insights Inc., Cascade, Colo.). Important features when designing oligonucleotides to be used as amplification primers include, but are not limited to, an appropriate size amplification product to facilitate detection (e.g., by electrophoresis), similar melting temperatures for the members of a pair of primers, and the length of each primer (i.e., the primers need to be long enough to anneal with sequence-specificity and to initiate synthesis but not so long that fidelity is reduced during oligonucleotide synthesis). Typically, oligonucleotide primers are 8 to 50 nucleotides in length (e.g., 8, 10, 12, 14, 16, 18, 20, 22, 24, 26, 28, 30, 32, 34, 36, 38, 40, 42, 44, 46, 48, or 50 nucleotides in length). In some embodiments oligonucleotide primers are 40 or fewer nucleotides in length. In addition to a set of primers, the methods may use one or more probes in order to detect the presence or absence of TV. The term "probe" refers to synthetically or biologically produced nucleic acids (DNA or RNA), which by design or selection, contain specific nucleotide sequences that allow them to hybridize under defined predetermined stringencies specifically (i.e., preferentially) to "target nucleic acids", in the present case to a target TV gene nucleic acid. A "probe" can be referred to as a "detection probe" meaning that it detects the target nucleic acid.

In some embodiments, the described target TV gene probes can be labeled with at least one fluorescent label. In one embodiment, the target TV gene probes can be labeled with a donor fluorescent moiety, e.g., a fluorescent dye, and a corresponding acceptor moiety, e.g., a quencher. In one embodiment, the probe comprises or consists of a fluorescent moiety and the nucleic acid sequences comprise or consist of SEQ ID NOs: 14-18.

Designing oligonucleotides to be used as probes can be performed in a manner similar to the design of primers. Embodiments may use a single probe or a pair of probes for detection of the amplification product. Depending on the embodiment, the probe(s) use may comprise at least one label and/or at least one quencher moiety. As with the primers, the probes usually have similar melting temperatures, and the length of each probe must be sufficient for sequence-specific hybridization to occur but not so long that fidelity is reduced during synthesis.

Oligonucleotide probes are generally 15 to 40 (e.g., 16, 18, 20, 21, 22, 23, 24, or 25) nucleotides in length.

Constructs can include vectors each containing one of target TV gene primers and probes nucleic acid molecules. Constructs can be used, for example, as control template nucleic acid molecules. Vectors suitable for use are commercially available and/or produced by recombinant nucleic acid technology methods routine in the art. Target TV gene nucleic acid molecules can be obtained, for example, by chemical synthesis, direct cloning from TV, or by PCR amplification.

Constructs suitable for use in the methods typically include, in addition to the target TV gene nucleic acid molecules sequences encoding a selectable marker (e.g., an antibiotic resistance gene) for selecting desired constructs and/or transformants, and an origin of replication. The choice of vector systems usually depends upon several factors, including, but not limited to, the choice of host cells, replication efficiency, selectability, inducibility, and the ease of recovery.

Constructs containing target TV gene nucleic acid molecules can be propagated in a host cell. As used herein, the term host cell is meant to include prokaryotes and eukaryotes such as yeast, plant and animal cells. Prokaryotic hosts may include *E. coli, Salmonella typhimurium, Serratia marcescens,* and *Bacillus subtilis.* Eukaryotic hosts include yeasts such as *S*. *cerevisiae, S. pombe, Pichia pastoris,* mammalian cells such as COS cells or Chinese hamster ovary (CHO) cells, insect cells, and plant cells such as *Arabidopsis thaliana* and *Nicotiana tabacum.* A construct can be introduced into a host cell using any of the techniques commonly known to those of ordinary skill in the art. For example, calcium phosphate precipitation, electroporation, heat shock, lipofection, microinjection, and viral-mediated nucleic acid transfer are common methods for introducing nucleic acids into host cells. In addition, naked DNA can be delivered directly to cells (see, e.g., U.S. Pat. Nos. 5,580,859 and 5,589,466).

### Detection of MG

The present disclosure provides methods to additionally detect MG in combination with TV by amplifying, for example, a portion of the target MG gene nucleic acid sequence. Nucleic acid sequences of the 23s ribosomal RNA gene, the mgpB gene and the MgPar partial repeats are publicly available (e.g., GenBank). Specifically, primers and probes to amplify and detect specific MG nucleic acid molecule targets are provided. For detection of MG, primers and probes to amplify the target MG gene are provided. Nucleic acids other than those exemplified herein can also be used to detect MG in a sample. For example, functional variants can be evaluated for specificity and/or sensitivity by those of skill in the art using routine methods. Representative functional variants can include, e.g., one or more deletions, insertions, and/or substitutions in the target MG gene nucleic acids disclosed herein.

More specifically, embodiments of the oligonucleotides each include a nucleic acid with a sequence selected from SEQ ID NOs: 106-194, a substantially identical variant thereof in which the variant has at least, e.g., 80%, 90%, or 95% sequence identity to one of SEQ ID NOs: 106-194, or a complement of SEQ ID NOs: 106-194 and the variant.

**TABLE I: 23s Forward Primers**

| **Forward Primers** | | | |
|---|---|---|---|
| **Oligo Name** | **SEQ ID NO:** | **Sequence** | **Modifications** |
| JH419BUA | 106 | GGGGTGGATCACCTCCTTTC<t_BB_dA> | t-butylbenzyldA |
| JH407BUC | 107 | CAATGTTTGGTCTCACAACTAACA<t_BB_dC> | t-butylbenzyldC |
| JH409BUA | 108 | TCCAGTTCTGAAAGAATGTTTTTGA<t_BB_dA> | t-butylbenzyldA |
| JH411BUA | 109 | AAACGACAATCTTTCTAGTTCCAAA<t_BB_dA> | t-butylbenzyldA |
| JH413BUC | 110 | ATGTTTGGTCTCACAACTAACA<t_BB_dC> | t-butylbenzyldC |
| JH415BUA | 111 | CAGTTCTGAAAGAATGTTTTTGA<t_BB_dA> | t-butylbenzyldA |
| JH417BUA | 112 | CGACAATCTTTCTAGTTCCAAA<t_BB_dA> | t-butylbenzyldA |

**TABLE II: 23s Reverse Primers**

| **Reverse Primers** | | | |
|---|---|---|---|
| **Oligo Name** | **SEQ ID NO:** | **Sequence** | **Modifications** |
| JH408BUC | 113 | CGGATCTCAGGTTTTTACCACCT<t_BB_dC> | t-butylbenzyldC |
| JH410BUA | 114 | CAGATTGCTCCATTCGGAC<t_BB_dA> | t-butylbenzyldA |
| JH412BUC | 115 | CAGATTGCTCCATTCGGACA<t_BB_dC> | t-butylbenzyldC |
| JH414BUC | 116 | AGATTGCTCCATTCGGACA<t_BB_dC> | t-butylbenzyldC |
| JH416BUA | 117 | CACGTCCTTCATCGCCTTTT<t_BB_dA> | t-butylbenzyldA |
| JH418BUC | 118 | GATCTCAGGTTTTTACCACCT<t_BB_dC> | t-butylbenzyldC |
| JH420BUA | 119 | ATTGCTCCATTCGGAC<t_BB_dA> | t-butylbenzyldA |
| JH422BUC | 120 | ATTGCTCCATTCGGACA<t_BB_dC> | t-butylbenzyldC |
| JH424BUC | 121 | ATTGCTCCATTCGGACA<t_BB_dC> | t-butylbenzyldC |
| JH426BUA | 122 | CGTCCTTCATCGCCTTTT<t_BB_dA> | t-butylbenzyldA |

**TABLE III: 23s Probes**

| **Probes** | | | |
|---|---|---|---|
| **Oligo Name** | **SEQ ID NO:** | **Sequence** | **Modifications** |
| JH437HQ6 | 123 | <H>GGTCAG<Q>TTTGTATCCAGTTCTGAAAGA ATGTTTTTGAAC<P> | P=phosphate, H=th-HEX, Q=BHQ2 |
| JH434HQ6 | 124 | <H>GTTCAA<Q>AAACATTCTTTCAGAACTGGA TACAAACTGACC<P> | P=phosphate, H=th-HEX, Q=BHQ2 |
| JH439HQ6 | 125 | <H>GAATGT<Q>TTTTGAACAGTTCTTTCAAAA CTGAAAACGACA<P> | P=phosphate, H=th-HEX, Q=BHQ2 |
| JH449HQ6 | 126 | <H>TCAGTT<Q>TGTATCCAGTTCTGAAAGAAT GTTTTTGAACCAG<P> | P=phosphate, H=th-HEX, Q=BHQ2 |
| JH438HQ6 | 127 | <H>TGTTCA<Q>AAAACATTCTTTCAGAACTGG ATACAAACTGACC<P> | P=phosphate, H=th-HEX, Q=BHQ2, |
| JH451HQ6 | 128 | <H>AGAATG<Q>TTTTTGAACAGTTCTTTCAAA ACTGAAAACGACA<P> | P=phosphate, H=th-HEX, Q=BHQ2 |
| JH441HQ6 | 129 | <H>CTAAAA<Q>GGCGATGAAGGACGTGTTAA CCTG<P> | P=phosphate, H=th-HEX, Q=BHQ2 |
| JH443HQ6 | 130 | <H>GGTCAG<Q>TTTGTATCCAGTTCTGAAAGA ATG<P> | P=phosphate, H=th-HEX, Q=BHQ2 |
| JH436HQ6 | 131 | <H>GTTCAA<Q>AAACATTCTTTCAGAACTGGA TACA<P> | P=phosphate, H=th-HEX, Q=BHQ2 |
| JH445HQ6 | 132 | <H>GAATGT<Q>TTTTGAACAGTTCTTTCAAAA CTGA<P> | P=phosphate, H=th-HEX, Q=BHQ2 |
| JH447HQ6 | 133 | <H>CTAAAA<Q>GGCGATGAAGGACGTGTTAA C<P> | P=phosphate, H=th-HEX, Q=BHQ2 |

**TABLE IV: mgpB Forward Primers**

| **Forward Primers** | | | |
|---|---|---|---|
| **Oligo Name** | **SEQ ID NO:** | **Sequence** | **Modifications** |
| JH311BUC | 134 | GACTTGAAACAATAACAACTTCTCTT<t_BB_dC> | t-butylbenzyldC |
| JH313BUA | 135 | GACTTGAAACAATAACAACTTCTCTTC<t_BB_dA> | t-butylbenzyldA |
| JH315BUA | 136 | AACAATAACAACTTCTCTTCACTAAAG<t_BB_dA> | t-butylbenzyldA |
| JH317BUA | 137 | CAATAACAACTTCTCTTCACTAAAGATT<t_BB_dA> | t-butylbenzyldA |
| JH319BUA | 138 | ACCCCTTGGACTTGAAACAATAACA<t_BB_dA> | t-butylbenzyldA |
| JH321BUA | 139 | AGAGAACCCAGGATCATTTGG<t_BB_dA> | t-butylbenzyldA |
| JH323BUA | 140 | CTGGAGAGAACCCAGGATC<t_BB_dA> | t-butylbenzyldA |

**TABLE V: mgpB Reverse Primers**

| **Reverse Primers** | | | |
|---|---|---|---|
| **Oligo Name** | **SEQ ID NO:** | **Sequence** | **Modifications** |
| JH310BUA | 141 | GTTGTTATCATACCTTCTCATTGCA<t_BB_dA> | t -butylbenzyldA |
| JH312BUA | 142 | CTACCGTTGTTATCATACCTTCTG<t_BB_dA> | t-butylbenzyldA |
| JH314BUA | 143 | CATATAAAGCTCTACCGTTGTTATCAT<t_BB_dA> | t -butylbenzyldA |
| JH316BUA | 144 | AATATCATATAAAGCTCTACCGTTGTT<t_BB_dA> | t -butylbenzyldA |
| JH320BUA | 145 | TTTTCCATTTTTGCTAAGTTAATATCATATA<t_BB_d A> | t-butylbenzyldA |
| JH322BUA | 146 | GGGGTTTTCCATTTTTGCTAAGTTA<t_BB_dA> | t -butylbenzyldA |

**TABLE VI: mgpB Probes**

| **Probes** | | | |
|---|---|---|---|
| **Oligo Name** | **SEQ ID NO:** | **Sequence** | **Modifications** |
| JH335HQ6 | 147 | <H>GGAGAG<Q>AACCCAGGATCATTTGGATT AGTAAGAAGC<P> | P=phosphate, H=th-HEX, Q=BHQ2 |
| JH337HQ6 | 148 | <H>AAGATT<Q>ACTGGAGAGAACCCAGGATC ATTTGGATTAGTAAG<P> | P=phosphate, H=th-HEX, Q=BHQ2 |
| JH339HQ6 | 149 | <H>CTGGAG<Q>AGAACCCAGGATCATTTGGA TTAGTAAGAAG<P> | P=phosphate, H=th-HEX, Q=BHQ2 |
| JH341HQ6 | 150 | <H>CAGCAA<Q>AACTTTGCAATCAGAAGGTAT GATAACAACG<P> | P=phosphate, H=th-HEX, Q=BHQ2 |
| JH342HQ6 | 151 | <H>CGTTGT<Q>TATCATACCTTCTGATTGCAA AGTTTTGCTG<P> | P=phosphate, H=th-HEX, Q=BHQ2 |

**TABLE VII: MgPar Forward Primers**

| **Forward Primers** | | | |
|---|---|---|---|
| **Oligo Name** | **SEQ ID NO:** | **Sequence** | **Modifications** |
| JH501BUA | 152 | TTTCTCCCCTGAATCGGCA<t_BB_dA> | t-butylbenzyldA |
| JH503BUA | 153 | CAACTCCCCCTCCCCTTCA<t_BB_dA> | t-butylbenzyldA |
| JH505BUC | 154 | TCCCCCTCCCCTTCAACTT<t_BB_dC> | t-butylbenzyldC |
| JH507BUC | 155 | ATCCCAATTCAGATGATAATAAAGTCA<t_BB_dC> | t-butylbenzyldC |
| JH509BUA | 156 | ATCCCAATTCAGATGATAATAAAGTC<t_BB_dA> | t-butylbenzyldA |
| JH511BUA | 157 | TCCCACCAGTGACTGGATCA<t_BB_dA> | t-butylbenzyldA |
| JLH531 | 158 | CAACTCCCACACTGCTTCC<t_BB_dC> | t-butylbenzyldC |
| KMMGP560F | 159 | TCCAACTCCCACACTGCTTCCC<t_BB_dC> | t-butylbenzyldC |
| KMMGP562F | 160 | TCCAACTCCCACACTGCTTC<t_BB_dC> | t-butylbenzyldC |
| KMMGP564F | 161 | CAACTCCCACACTGCTTC<t_BB_dC> | t-butylbenzyldC |

**TABLE VIII: MgPar Reverse Primers**

| **Reverse Primers** | | | |
|---|---|---|---|
| **Oligo Name** | **SEQ ID NO:** | **Sequence** | **Modifications** |
| JH502BUA | 162 | GGTGAAAAGTTAGGTATAAACACCC<t_BB_dA > | t-butylbenzyldA |
| JH504BUA | 163 | CTGCTCCTGTTCAGATGTC<t_BB_dA> | t-butylbenzyldA |
| JH506BUA | 164 | TGCTCACTATCCTTGTTAAATTG<t_BB_dA> | t-butylbenzyldA |
| JH508BUA | 165 | CACCTCCCCAAACCCAGGTAA<t_BB_dA> | t-butylbenzyldA |
| JH510BUA | 166 | ACCTCCCCAAACCCAGGTAA<t_BB_dA> | t-butylbenzyldA |
| JLH536 | 167 | CCTGCTCCCGTTCAGATGT<t_BB_dC> | t-butylbenzyldC |
| JLH540 | 168 | CCTGCTCCCGTT<L>AAATGT<t_BB_dC> | t-butylbenzyldC, L=9-(aminoethoxy)-phenoxazine-2' -dC |
| JLH538R | 169 | CCTGCTCCCGTTCA<R>ATGT<t_BB_dC> | t-butylbenzyldC R= A or G |
| JLH538R_A | 170 | CCTGCTCCCGTTCAAATGT<t_BB_dC> | t-butylbenzyldC |
| JLH538R_G | 171 | CCTGCTCCCGTTCAGATGT<t_BB_dC> | t -butylbenzyldC |

**TABLE IX: MgPar Probes**

| **Probes** | | | |
|---|---|---|---|
| **Oligo Name** | **SEQ ID NO:** | **Sequence** | **Modifications** |
| JH521HQ6 | 172 | <H>CTCCCC<Q>ACTTTTTCTAACAT CAATGTTGGGGTTAAATCAA<P> | P=phosphate, H=th-HEX, Q=BHQ2 |
| JH522HQ6 | 173 | <H>TTGATT<Q>TAACCCCAACATTG ATGTTAGAAAAAGTGGGGAG<P> | P=phosphate, H=th-HEX, Q=BHQ2 |
| JH523HQ6 | 174 | <H>CGCAAT<Q>CAACTGTTGCTCAG AAGCTTACTAGGAA<P> | P=phosphate, H=th-HEX, Q=BHQ2 |
| JH524HQ6 | 175 | <H>AGTTCC<Q>TAGTAAGCTTCTGA GCAACAGTTGATTGC<P> | P=phosphate, H=th-HEX, Q=BHQ2 |
| JLH540HQ6 | 176 | <H>GATTTA<Q>ACCCCAACATTGAT GTTAGAAAAAGTGGGGAG<P> | P=phosphate, H=th-HEX, Q=BHQ2 |
| JLH540PDUHQ6 | 177 | <H>GA<U><U><U>A<Q>ACCCCAAC A<U><U>GA<U>G<U><U>AGAAAAA G<U>GGGAG<P> | P=phosphate, H=th-HEX, Q=BHQ2, U=propynyldU |
| JLH544HQ6 | 178 | <H>ATTTAA<Q>CCCCAACATTGATG TTAGAAAAAGTGGGGAG<P> | P=phosphate, H=th-HEX, Q=BHQ2 |
| JLH544PDUHQ6 | 179 | <H>A<U><U><U>AA<Q>CCCCAACA< U><U>GA<U>G<U><U>AGAAAAG< U>GGGGAG<P> | P=phosphate, H=th-HEX, Q=BHQ2, U=propynyldU |
| JLH546HQ6 | 180 | <H>GATTTA<Q>ACCCCAACATTGAT GTTAGAAAAAGTGGTGAG<P> | P=phosphate, H=th-HEX, Q=BHQ2 |
| JLH546PDUHQ6 | 181 | <H>GATTTA<Q>ACCCCAACA<U><U >GA<U>G<U><U>AGAAAAG<U>GG TGAG<P> | P=phosphate, H=th-HEX, Q=BHQ2, U=propynyldU |
| JLH548HQ6 | 182 | <H>ATTTAA<Q>CCCCAACATTGATG TTAGAAAAAGTGGTGAG<P> | P=phosphate, H=th-HEX, Q=BHQ2 |
| JLH548PDUHQ6 | 183 | <H>ATTTAA<Q>CCCCAACA<U><U> GA<U>G<U><U>AGAAAAG<U>GGT GAG<P> | P=phosphate, H=th-HEX, Q=BHQ2, U=propynyldU |
| JLH550HQ6 | 184 | <H>ATTTAA<Q>CCCCAACATTGATG TTAGAAAAAGT<Y>GGGAG<P> | P=phosphate, H=th-HEX, Q=BHQ2, Y=7' deazadG |
| JLH552HQ6 | 185 | <H>ATTTAA<Q>CCCCAACATTGATG TTAGAAAAAGTG<Y>GGAG<P> | P=phosphate, H=th-HEX, Q=BHQ2, Y=7' deazadG |
| JLH554HQ6 | 186 | <H>ATTTAA<Q>CCCCAACATTGATG TTAGAAAAAGTGG<Y>GAG<P> | P=phosphate, H=th-HEX, Q=BHQ2, Y=7' deazadG |
| JLH556HQ6 | 187 | <H>ATTTAA<Q>CCCCAACATTGATG TTAGAAAAAGTGGG<Y>AG<P> | P=phosphate, H=th-HEX, Q=BHQ2, Y=7' deazadG |
| JLH550PDUHQ6 | 188 | <H>A<U><U><U>AA<Q>CCCCAACA< U><U>GA<U>G<U><U>AGAAAAAG< U><Y>GGGAG<P> | P=phosphate, H=th-HEX, Q=BHQ2, U=propynyldU Y=7' deazadG |
| JLH552PDUHQ6 | 189 | <H>A<U><U><U>AA<Q>CCCCAACA< U><U>GA<U>G<U><U>AGAAAAAG< U>G<Y>GGAG<P> | P=phosphate, H=th-HEX, Q=BHQ2, U=propynyldU Y=7' deazadG |
| JLH554PDUHQ6 | 190 | <H>A<U><U><U>AA<Q>CCCCAACA< U><U>GA<U>G<U><U>AGAAAAAG< U>GG<Y>GAG<P> | P=phosphate, H=th-HEX, Q=BHQ2, U=propynyldU Y=7' deazadG |
| JLH558HQ6 | 191 | <H>ATTTAA<Q>CCCCAACATTGATG TTAGAAAAAGTG<Y>G<P> | P=phosphate, H=th-HEX, Q=BHQ2, Y=7' deazadG |
| KMMGP560HQ6 | 192 | <H>ATTTAA<Q>CCCCAACATTGATG TTAGAAAAAGTGGG<P> | P=phosphate, H=th-HEX, Q=BHQ2 |
| KMMGP562HQ6 | 193 | <H>A<U><U><U>AA<Q>CCCCAACA< U><U>GA<U>G<U><U>AGAAAAGT GGG<P> | P=phosphate, H=th-HEX, Q=BHQ2, U=propynyldU |
| KMMGP564HQ6 | 194 | <H>A<U><U><U>AA<Q>C<X>C<X>A A<X>A<U><U>GA<U>G<U><U>AGAA AAAGTGGG<P> | P=phosphate, H=th-HEX, Q=BHQ2, U=propynyldU X=propynyldC |

In one embodiment, the above described sets of primers and probes are used in order to provide for the additional detection of MG in a biological sample suspected of containing MG. The sets of primers and probes may comprise or consist of the primers and probes specific for the nucleic acid sequences of the 23s gene, mgpB gene, and the MgPar partial repeats comprising or consisting of the nucleic acid sequences of SEQ ID NOs: 106-194. In another embodiment, the primers and probes for the target MG genes comprise or consist of a functionally active variant of any of the primers and probes of SEQ ID NOs: 106-194.

A functionally active variant of any of the primers and/or probes of SEQ ID NOs: 106-194 may be identified by using the primers and/or probes in the disclosed methods. A functionally active variant of a primer and/or probe of any of the SEQ ID NOs: 106-194 pertains to a primer and/or probe which provides a similar or higher specificity and sensitivity in the described method or kit as compared to the respective sequence of SEQ ID NOs: 106-194.

The variant may, e.g., vary from the sequence of SEQ ID NOs: 106-194 by one or more nucleotide additions, deletions or substitutions such as one or more nucleotide additions, deletions or substitutions at the 5' end and/or the 3' end of the respective sequence of SEQ ID NOs: 106-194. As detailed above, a primer (and/or probe) may be chemically modified, i.e., a primer and/or probe may comprise a modified nucleotide or a non-nucleotide compound. A probe (or a primer) is then a modified oligonucleotide. "Modified nucleotides" (or "nucleotide analogs") differ from a natural "nucleotide" by some modification but still consist of a base or base-like compound, a pentofuranosyl sugar or a pentofuranosyl sugar-like compound, a phosphate portion or phosphate-like portion, or combinations thereof. For example, a "label" may be attached to the base portion of a "nucleotide" whereby a "modified nucleotide" is obtained. A natural base in a "nucleotide" may also be replaced by, e.g., a 7-deazapurine whereby a "modified nucleotide" is obtained as well. The terms "modified nucleotide" or "nucleotide analog" are used interchangeably in the present application. A "modified nucleoside" (or "nucleoside analog") differs from a natural nucleoside by some modification in the manner as outlined above for a "modified nucleotide" (or a "nucleotide analog").

Oligonucleotides including modified oligonucleotides and oligonucleotide analogs that amplify a nucleic acid molecule encoding the target MG gene, e.g. the 23s gene, can be designed using, for example, a computer program such as OLIGO (Molecular Biology Insights Inc., Cascade, Colo.). Important features when designing oligonucleotides to be used as amplification primers include, but are not limited to, an appropriate size amplification product to facilitate detection (e.g., by electrophoresis), similar melting temperatures for the members of a pair of primers, and the length of each primer (i.e., the primers need to be long enough to anneal with sequence-specificity and to initiate synthesis but not so long that fidelity is reduced during oligonucleotide synthesis). Typically, oligonucleotide primers are 8 to 50 nucleotides in length (e.g., 8, 10, 12, 14, 16, 18, 20, 22, 24, 26, 28, 30, 32, 34, 36, 38, 40, 42, 44, 46, 48, or 50 nucleotides in length).

In addition to a set of primers, the methods may use one or more probes in order to detect the presence or absence of MG. The term "probe" refers to synthetically or biologically produced nucleic acids (DNA or RNA), which by design or selection, contain specific nucleotide sequences that allow them to hybridize under defined predetermined stringencies specifically (i.e., preferentially) to "target nucleic acids", in the present case to a target MG gene nucleic acid. A "probe" can be referred to as a "detection probe" meaning that it detects the target nucleic acid.

In some embodiments, the described target MG gene probes can be labeled with at least one fluorescent label. In one embodiment, the target MG gene probes can be labeled with a donor fluorescent moiety, e.g., a fluorescent dye, and a corresponding acceptor moiety, e.g., a quencher. In one embodiment, the probe comprises or consists of a fluorescent moiety and the nucleic acid sequences comprise or consist of SEQ ID NOs: 123-133, 147-151, and 172-194. Designing oligonucleotides to be used as probes can be performed in a manner similar to the design of primers. Embodiments may use a single probe or a pair of probes for detection of the amplification product. Depending on the embodiment, the probe(s) use may comprise at least one label and/or at least one quencher moiety. As with the primers, the probes usually have similar melting temperatures, and the length of each probe must be sufficient for sequence-specific hybridization to occur but not so long that fidelity is reduced during synthesis. Oligonucleotide probes are generally 15 to 40 (e.g., 16, 18, 20, 21, 22, 23, 24, or 25) nucleotides in length. In some embodiments oligonucleotide primers are 40 or fewer nucleotides in length.

### Polymerase Chain Reaction (PCR)

U.S. Pat. Nos. 4,683,202, 4,683,195, 4,800,159, and 4,965,188 disclose conventional PCR techniques. PCR typically employs two oligonucleotide primers that bind to a selected nucleic acid template (e.g., DNA or RNA). Primers useful in some embodiments include oligonucleotides capable of acting as points of initiation of nucleic acid synthesis within the described target TV gene nucleic acid sequences (e.g., SEQ ID NOs: 1-13, 19-38, 45-52, 55-74, and 80-99). A primer can be purified from a restriction digest by conventional methods, or it can be produced synthetically. The primer is preferably single-stranded for maximum efficiency in amplification, but the primer can be double-stranded. Double-stranded primers are first denatured, i.e., treated to separate the strands. One method of denaturing double stranded nucleic acids is by heating.

If the template nucleic acid is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (e.g., greater than 50%, 60%, 70%, 80%, 90% or 95% denatured). The heating conditions necessary for denaturing template nucleic acid will depend, e.g., on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 30 sec to 4 min (e.g., 1 min to 2 min 30 sec, or 1.5 min).

If the double-stranded template nucleic acid is denatured by heat, the reaction mixture is allowed to cool to a temperature that promotes annealing of each primer to its target sequence on the described target TV gene nucleic acid molecules. The temperature for annealing is usually from about 35°C to about 65°C (e.g., about 40°C to about 60°C; about 45°C to about 50°C). Annealing times can be from about 10 sec to about 1 min (e.g., about 20 sec to about 50 sec; about 30 sec to about 40 sec). The reaction mixture is then adjusted to a temperature at which the activity of the polymerase is promoted or optimized, i.e., a temperature sufficient for extension to occur from the annealed primer to generate products complementary to the template nucleic acid. The temperature should be sufficient to synthesize an extension product from each primer that is annealed to a nucleic acid template, but should not be so high as to denature an extension product from its complementary template (e.g., the temperature for extension generally ranges from about 40°C to about 80°C (e.g., about 50°C to about 70°C; about 60°C). Extension times can be from about 10 sec to about 5 min (e.g., about 30 sec to about 4 min; about 1 min to about 3 min; about 1 min 30 sec to about 2 min).

PCR assays can employ nucleic acid such as RNA or DNA (cDNA). The template nucleic acid need not be purified; it may be a minor fraction of a complex mixture, such as nucleic acid contained in human cells. Nucleic acid molecules may be extracted from a biological sample by routine techniques such as those described in Diagnostic Molecular Microbiology: Principles and Applications (Persing et al. (eds), 1993, American Society for Microbiology, Washington D.C.). Nucleic acids can be obtained from any number of sources, such as plasmids, or natural sources including bacteria, yeast, protozoa viruses, organelles, or higher organisms such as plants or animals.

The oligonucleotide primers are combined with PCR reagents under reaction conditions that induce primer extension. For example, chain extension reactions generally include 50 mM KCl, 10 mM Tris-HCl (pH 8.3), 15 mM MgCl2, 0.001% (w/v) gelatin, 0.5-1.0 µg protodenatured template DNA, 50 pmoles of each oligonucleotide primer, 2.5 U of Taq polymerase, and 10% DMSO). The reactions usually contain 150 to 320 µM each of dATP, dCTP, dTTP, dGTP, or one or more analogs thereof.

The newly synthesized strands form a double-stranded molecule that can be used in the succeeding steps of the reaction. The steps of strand separation, annealing, and elongation can be repeated as often as needed to produce the desired quantity of amplification products corresponding to the target nucleic acid molecules. The limiting factors in the reaction are the amounts of primers, thermostable enzyme, and nucleoside triphosphates present in the reaction. The cycling steps (i.e., denaturation, annealing, and extension) are preferably repeated at least once. For use in detection, the number of cycling steps will depend, e.g., on the nature of the sample. If the sample is a complex mixture of nucleic acids, more cycling steps will be required to amplify the target sequence sufficient for detection. Generally, the cycling steps are repeated at least about 20 times, but may be repeated as many as 40, 60, or even 100 times.

### Fluorescence Resonance Energy Transfer (FRET)

FRET technology (see, for example, U.S. Pat. Nos. 4,996,143, 5,565,322, 5,849,489, and 6,162,603) is based on a concept that when a donor fluorescent moiety and a corresponding acceptor fluorescent moiety are positioned within a certain distance of each other, energy transfer takes place between the two fluorescent moieties that can be visualized or otherwise detected and/or quantitated. The donor typically transfers the energy to the acceptor when the donor is excited by light radiation with a suitable wavelength. The acceptor typically re-emits the transferred energy in the form of light radiation with a different wavelength. In certain systems, non-fluorescent energy can be transferred between donor and acceptor moieties, by way of biomolecules that include substantially non-fluorescent donor moieties (see, for example, US Pat. No. 7,741,467).

In one example, a oligonucleotide probe can contain a donor fluorescent moiety and a corresponding quencher, which may or not be fluorescent, and which dissipates the transferred energy in a form other than light. When the probe is intact, energy transfer typically occurs between the donor and acceptor moieties such that fluorescent emission from the donor fluorescent moiety is quenched the acceptor moiety. During an extension step of a polymerase chain reaction, a probe bound to an amplification product is cleaved by the 5' to 3' nuclease activity of, e.g., a Taq Polymerase such that the fluorescent emission of the donor fluorescent moiety is no longer quenched. Exemplary probes for this purpose are described in, e.g., U.S. Pat. Nos. 5,210,015, 5,994,056, and 6,171,785. Commonly used donor-acceptor pairs include the FAM-TAMRA pair. Commonly used quenchers are DABCYL and TAMRA. Commonly used dark quenchers include BlackHole Quenchers™ (BHQ), (Biosearch Technologies, Inc., Novato, Cal.), Iowa Black™, (Integrated DNA Tech., Inc., Coralville, Iowa), BlackBerry™ Quencher 650 (BBQ-650), (Berry & Assoc., Dexter, Mich.).

In another example, two oligonucleotide probes, each containing a fluorescent moiety, can hybridize to an amplification product at particular positions determined by the complementarity of the oligonucleotide probes to the target nucleic acid sequence. Upon hybridization of the oligonucleotide probes to the amplification product nucleic acid at the appropriate positions, a FRET signal is generated. Hybridization temperatures can range from about 35° C. to about 65° C. for about 10 sec to about 1 min.

Fluorescent analysis can be carried out using, for example, a photon counting epifluorescent microscope system (containing the appropriate dichroic mirror and filters for monitoring fluorescent emission at the particular range), a photon counting photomultiplier system, or a fluorimeter. Excitation to initiate energy transfer, or to allow direct detection of a fluorophore, can be carried out with an argon ion laser, a high intensity mercury (Hg) arc lamp, a fiber optic light source, or other high intensity light source appropriately filtered for excitation in the desired range.

As used herein with respect to donor and corresponding acceptor moieties "corresponding" refers to an acceptor fluorescent moiety or a dark quencher having an absorbance spectrum that overlaps the emission spectrum of the donor fluorescent moiety. The wavelength maximum of the emission spectrum of the acceptor fluorescent moiety should be at least 100 nm greater than the wavelength maximum of the excitation spectrum of the donor fluorescent moiety. Accordingly, efficient non-radiative energy transfer can be produced there between.

Fluorescent donor and corresponding acceptor moieties are generally chosen for (a) high efficiency Forster energy transfer; (b) a large final Stokes shift (>100 nm); (c) shift of the emission as far as possible into the red portion of the visible spectrum (>600 nm); and (d) shift of the emission to a higher wavelength than the Raman water fluorescent emission produced by excitation at the donor excitation wavelength. For example, a donor fluorescent moiety can be chosen that has its excitation maximum near a laser line (for example, Helium-Cadmium 442 nm or Argon 488 nm), a high extinction coefficient, a high quantum yield, and a good overlap of its fluorescent emission with the excitation spectrum of the corresponding acceptor fluorescent moiety. A corresponding acceptor fluorescent moiety can be chosen that has a high extinction coefficient, a high quantum yield, a good overlap of its excitation with the emission of the donor fluorescent moiety, and emission in the red part of the visible spectrum (>600 nm). Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothio-cyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC Red 640, LC Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate, or other chelates of Lanthanide ions (e.g., Europium, or Terbium). Donor and acceptor fluorescent moieties can be obtained, for example, from Molecular Probes (Junction City, Oreg.) or Sigma Chemical Co. (St. Louis, Mo.).

The donor and acceptor fluorescent moieties can be attached to the appropriate probe oligonucleotide via a linker arm. The length of each linker arm is important, as the linker arms will affect the distance between the donor and acceptor fluorescent moieties. The length of a linker arm can be the distance in Angstroms (Å) from the nucleotide base to the fluorescent moiety. In general, a linker arm is from about 10 Å to about 25 Å. The linker arm may be of the kind described in WO 84/03285. WO 84/03285 also discloses methods for attaching linker arms to a particular nucleotide base, and also for attaching fluorescent moieties to a linker arm.

An acceptor fluorescent moiety, such as an LC Red 640, can be combined with an oligonucleotide which contains an amino linker (e.g., C6-amino phosphoramidites available from ABI (Foster City, Calif.) or Glen Research (Sterling, VA)) to produce, for example, LC Red 640-labeled oligonucleotide. Frequently used linkers to couple a donor fluorescent moiety such as fluorescein to an oligonucleotide include thiourea linkers (FITC-derived, for example, fluorescein-CPG's from Glen Research or ChemGene (Ashland, Mass.)), amide-linkers (fluorescein-NHS-ester-derived, such as CX-fluorescein-CPG from BioGenex (San Ramon, Calif.)), or 3'-amino-CPGs that require coupling of a fluorescein-NHS-ester after oligonucleotide synthesis.

### Detection of TV and Detection of TV and MG

The present disclosure provides methods for detecting the presence or absence of TV and methods for detecting the presence or absence of TV and/or MG in a biological or non-biological sample. Methods provided avoid problems of sample contamination, false negatives, and false positives. The methods include performing at least one cycling step that includes amplifying a portion of target nucleic acid molecules from a sample using one or more pairs of primers, and a FRET detecting step. Multiple cycling steps are performed, preferably in a thermocycler. Methods can be performed using the primers and probes to detect the presence of TV, and the detection of the target TV gene indicates the presence of TV in the sample. In other embodiments methods can be performed using the primers and probes to detect the presence of TV and/or MG, and the detection of the target TV and/or MG gene indicates the presence of TV and/or MG in the sample.

As described herein, amplification products can be detected using labeled hybridization probes that take advantage of FRET technology. One FRET format utilizes TaqMan® technology to detect the presence or absence of an amplification product, and hence, the presence or absence of TV. TaqMan^{®} technology utilizes one single-stranded hybridization probe labeled with, e.g., one fluorescent dye and one quencher, which may or may not be fluorescent. When a first fluorescent moiety is excited with light of a suitable wavelength, the absorbed energy is transferred to a second fluorescent moiety or a dark quencher according to the principles of FRET. The second moiety is generally a quencher molecule. During the annealing step of the PCR reaction, the labeled hybridization probe binds to the target DNA (i.e., the amplification product) and is degraded by the 5' to 3' nuclease activity of, e.g., the Taq Polymerase during the subsequent elongation phase. As a result, the fluorescent moiety and the quencher moiety become spatially separated from one another. As a consequence, upon excitation of the first fluorescent moiety in the absence of the quencher, the fluorescence emission from the first fluorescent moiety can be detected. By way of example, an ABI PRISM® 7700 Sequence Detection System (Applied Biosystems) uses TaqMan® technology, and is suitable for performing the methods described herein for detecting the presence or absence of TV in the sample and detecting the presence or absence of TV and/or MG in the sample.

Molecular beacons in conjunction with FRET can also be used to detect the presence of an amplification product using the real-time PCR methods. Molecular beacon technology uses a hybridization probe labeled with a first fluorescent moiety and a second fluorescent moiety. The second fluorescent moiety is generally a quencher, and the fluorescent labels are typically located at each end of the probe. Molecular beacon technology uses a probe oligonucleotide having sequences that permit secondary structure formation (e.g., a hairpin). As a result of secondary structure formation within the probe, both fluorescent moieties are in spatial proximity when the probe is in solution. After hybridization to the target nucleic acids (i.e., amplification products), the secondary structure of the probe is disrupted and the fluorescent moieties become separated from one another such that after excitation with light of a suitable wavelength, the emission of the first fluorescent moiety can be detected.

Another common format of FRET technology utilizes two hybridization probes. Each probe can be labeled with a different fluorescent moiety and are generally designed to hybridize in close proximity to each other in a target DNA molecule (e.g., an amplification product). A donor fluorescent moiety, for example, fluorescein, is excited at 470 nm by the light source of the LightCycler® Instrument. During FRET, the fluorescein transfers its energy to an acceptor fluorescent moiety such as LightCycler®-Red 640 (LC Red 640) or LightCycler®-Red 705 (LC Red 705). The acceptor fluorescent moiety then emits light of a longer wavelength, which is detected by the optical detection system of the LightCycler® instrument. Efficient FRET can only take place when the fluorescent moieties are in direct local proximity and when the emission spectrum of the donor fluorescent moiety overlaps with the absorption spectrum of the acceptor fluorescent moiety. The intensity of the emitted signal can be correlated with the number of original target DNA molecules (e.g., the number of TV genomes). If amplification of target nucleic acid occurs and an amplification product is produced, the step of hybridizing results in a detectable signal based upon FRET between the members of the pair of probes. Generally, the presence of FRET indicates the presence of TV in the sample, and the absence of FRET indicates the absence of TV in the sample. Inadequate specimen collection, transportation delays, inappropriate transportation conditions, or use of certain collection swabs (calcium alginate or aluminum shaft) are all conditions that can affect the success and/or accuracy of a test result, however. Using the methods disclosed herein, detection of FRET within, e.g., 45 cycling steps is indicative of a TV infection. In other embodiments, detection of FRET within, e.g., 45 cycling steps is indicative of a TV and/or MG infection.

Representative biological samples that can be used in practicing the methods include, but are not limited to respiratory specimens, fecal specimens, blood specimens, dermal swabs, nasal swabs, wound swabs, blood cultures, skin, and soft tissue infections. Collection and storage methods of biological samples are known to those of skill in the art. Biological samples can be processed (e.g., by nucleic acid extraction methods and/or kits known in the art) to release TV nucleic acid or in some cases, the biological sample can be contacted directly with the PCR reaction components and the appropriate oligonucleotides. Accordingly, biological samples can be processed (e.g., by nucleic acid extraction methods and/or kits known in the art) to release TV and/or MG nucleic acid(s) or in some cases, the biological sample can be contacted directly with the PCR reaction components and the appropriate oligonucleotides.

Melting curve analysis is an additional step that can be included in a cycling profile. Melting curve analysis is based on the fact that DNA melts at a characteristic temperature called the melting temperature (Tm), which is defined as the temperature at which half of the DNA duplexes have separated into single strands. The melting temperature of a DNA depends primarily upon its nucleotide composition. Thus, DNA molecules rich in G and C nucleotides have a higher Tm than those having an abundance of A and T nucleotides. By detecting the temperature at which signal is lost, the melting temperature of probes can be determined. Similarly, by detecting the temperature at which signal is generated, the annealing temperature of probes can be determined. The melting temperature(s) of the probes from the amplification products can confirm the presence or absence of TV in the sample. Accordingly, the melting temperature(s) of the probes from the amplification products can confirm the presence or absence of TV and/or MG in the sample.

Within each thermocycler run, control samples can be cycled as well. Positive control samples can amplify target nucleic acid control template (other than described amplification products of target genes) using, for example, control primers and control probes. Positive control samples can also amplify, for example, a plasmid construct containing the target nucleic acid molecules. Such a plasmid control can be amplified internally (e.g., within the sample) or in a separate sample run side-by-side with the patients' samples using the same primers and probe as used for detection of the intended target. Such controls are indicators of the success or failure of the amplification, hybridization, and/or FRET reaction. Each thermocycler run can also include a negative control that, for example, lacks target template DNA. Negative control can measure contamination. This ensures that the system and reagents would not give rise to a false positive signal. Therefore, control reactions can readily determine, for example, the ability of primers to anneal with sequence-specificity and to initiate elongation, as well as the ability of probes to hybridize with sequence-specificity and for FRET to occur.

In an embodiment, the methods include steps to avoid contamination. For example, an enzymatic method utilizing uracil-DNA glycosylase is described in U.S. Pat. Nos. 5,035,996, 5,683,896 and 5,945,313 to reduce or eliminate contamination between one thermocycler run and the next.

Conventional PCR methods in conjunction with FRET technology can be used to practice the methods. In one embodiment, a LightCycler^{®} instrument is used. The following patent applications describe real-time PCR as used in the LightCycler^{®} technology: WO 97/46707, WO 97/46714, and WO 97/46712.

The LightCycler^{®} can be operated using a PC workstation and can utilize a Windows NT operating system. Signals from the samples are obtained as the machine positions the capillaries sequentially over the optical unit. The software can display the fluorescence signals in real-time immediately after each measurement. Fluorescent acquisition time is 10-100 milliseconds (msec). After each cycling step, a quantitative display of fluorescence vs. cycle number can be continually updated for all samples. The data generated can be stored for further analysis.

As an alternative to FRET, an amplification product can be detected using a double-stranded DNA binding dye such as a fluorescent DNA binding dye (e.g., SYBR® Green or SYBR® Gold (Molecular Probes)). Upon interaction with the double-stranded nucleic acid, such fluorescent DNA binding dyes emit a fluorescence signal after excitation with light at a suitable wavelength.

A double-stranded DNA binding dye such as a nucleic acid intercalating dye also can be used.

When double-stranded DNA binding dyes are used, a melting curve analysis is usually performed for confirmation of the presence of the amplification product.

It is understood that the embodiments of the present disclosure are not limited by the configuration of one or more commercially available instruments.

### Articles of Manufacture/Kits

Embodiments of the present disclosure further provide for articles of manufacture, compositions or kits to detect TV. An article of manufacture can include primers and probes used to detect the target TV gene, together with suitable packaging materials. Compositions can include primers used to amplify the target TV gene. In certain embodiments compositions can also comprise probes for detecting the target TV gene. Representative primers and probes for detection of TV are capable of hybridizing to target nucleic acid molecules. In addition, the kits may also include suitably packaged reagents and materials needed for DNA immobilization, hybridization, and detection, such solid supports, buffers, enzymes, and DNA standards. Methods of designing primers and probes are disclosed herein, and representative examples of primers and probes that amplify and hybridize to target nucleic acid molecules are provided.

Articles of manufacture can also include one or more fluorescent moieties for labeling the probes or, alternatively, the probes supplied with the kit can be labeled. For example, an article of manufacture may include a donor and/or an acceptor fluorescent moiety for labeling the probes. Examples of suitable FRET donor fluorescent moieties and corresponding acceptor fluorescent moieties are provided above.

Articles of manufacture can also contain a package insert or package label having instructions thereon for using the primers and probes to detect TV in a sample. Articles of manufacture and compositions may additionally include reagents for carrying out the methods disclosed herein (e.g., buffers, polymerase enzymes, co-factors, or agents to prevent contamination). Such reagents may be specific for one of the commercially available instruments described herein. Embodiments of the present disclosure will be further described in the following examples, which do not limit the scope of the invention described in the claims.

### EXAMPLES

The following examples, tables and figures are provided to aid the understanding of the subject matter, the true scope of which is set forth in the appended claims. It is understood that modifications can be made in the procedures set forth without departing from the spirit of the invention.

### EXAMPLE I

Target selection for TV was the result of a comprehensive search of the public sequence database, as well as a literature search for TV targets with a potential to discriminate against the nearest neighbors, *Trichomonas tenax* and *Pentatrichomonas hominis.* Multiple targets from the public sequence database were analyzed in the target selection process of the design phase, but all showed cross reactivity with *T. tenax* and *P. hominis.* The sequences in the public database are complicated by "bulk" sequence data from multicopy targets. BLAST analysis of the chosen oligonucleotides indicated that the only significant cross reactivity will be with *Trichomonas tenax.*

Real-time PCR detection of TV were performed using either the **cobas**® 4800 system or the **cobas**® 6800/8800 systems platforms (Roche Molecular Systems, Inc., Pleasanton, CA). The final concentrations of the amplification reagents are shown below:

**TABLE XVI PCR Amplification Reagents**

| ***Master Mix Component*** | ***Final Cone (50uL)*** | |
|---|---|---|
| DMSO | 0-5.4 | % |
| NaN3 | 0.027-0.030 | % |
| Potassium acetate | 120.0 | mM |
| Glycerol | 3.0 | % |
| Tween 20 | 0.02 | % |
| EDTA | 0-43.9 | uM |
| Tricine | 60.0 | mM |
| Aptamer | 0.18-0.22 | uM |
| UNG Enzyme | 5.0-10.0 | U |
| Z05-SP-PZ Polymerase | 30.0-45.0 | U |
| dATP | 400.0-521.70 | uM |
| dCTP | 400.0-521.70 | uM |
| dGTP | 400.0-521.70 | uM |
| dUTP | 800.0-1043.40 | uM |
| Forward primer oligonucleotides | 0.15-0.50 | µM |
| Reverse primer oligonucleotides | 0.15-0.50 | µM |
| Probe oligonucleotides | 0.10 | µM |
| Manganese Acetate | 3.30-3.80 | mM |

The following table shows the typical thermoprofile used for PCR amplification reaction:

**TABLE XVII PCR Thermoprofile**

| Program Name | Target (°C) | Acquisition Mode | Hold (hh:mm:ss) | Ramp Rate (°C / s) | Cycles | Analysis Mode |
|---|---|---|---|---|---|---|
| Pre-PCR | 50 | None | 00:02:00 | 4.4 | 1 | None |
| | 94 | None | 00:00:05 | 4.4 | | |
| | 55 | None | 00:02:00 | 2.2 | | |
| | 60 | None | 00:06:00 | 4.4 | | |
| | 65 | None | 00:04:00 | 4.4 | | |
| 1st Measurement | | | | | 5 | Quantification |
| | 95 | None | 00:00:05 | 4.4 | | |
| | 55 | Single | 00:00:30 | 2.2 | | |
| 2nd Measurement | | | | | 45 | Quantification |
| | 91 | None | 00:00:05 | 4.4 | | |
| | 58 | Single | 00:00:25 | 2.2 | | |
| Cooling | 40 | None | 00:02:00 | 2.2 | 1 | None |

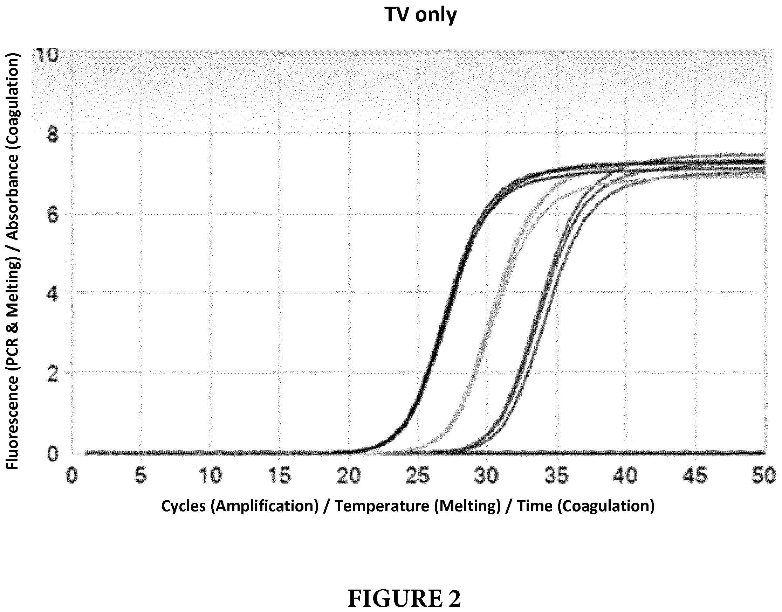

The Pre-PCR program comprised initial denaturing and incubation at 55°C, 60°C and 65°C for reverse transcription of RNA templates. Incubating at three temperatures combines the advantageous effects that at lower temperatures slightly mismatched target sequences (such as genetic variants of an organism) are also transcribed, while at higher temperatures the formation of RNA secondary structures is suppressed, thus leading to a more efficient transcription. PCR cycling was divided into two measurements, wherein both measurements apply a one-step setup (combining annealing and extension). The first 5 cycles at 55°C allow for an increased inclusivity by pre-amplifying slightly mismatched target sequences, whereas the 45 cycles of the second measurement provide for an increased specificity by using an annealing/extension temperature of 58°C. Figure 2 depicts a typical amplification experiment where PCR growth curves are shown in various concentrations of genomic TV template DNA. The amplification and detection of the target TV genes, 5.8s rRNA, 18s rRNA, PMS1, Mlhla and CRN were performed using the conditions described above. The results of the experiments using several selected oligonucleotide primers and probes against genomic TV DNA present at a concentration of either 10 genomic equivalent/PCR (5.8s rRNA) or 1000 genomic equivalent/PCR (18s rRNA, PMS1, Mlhla, CRN) are shown below as Ct values (threshold cycle) for the amplification reactions.

**TABLE XVIII Amplification and Detection of target TV genes**

| **Target TV Gene** | **Forward primer SEQ ID NO** | **Reverse primer SEQ ID NO** | **Probe SEQ ID NO** | **Ct values 10 ge/PCR of TV** |
|---|---|---|---|---|
| 5.8s rRNA | 3 | 12 | 16 | 27.3 |
| | 3 | 12 | 17 | 30.1 |
| | 3 | 12 | 18 | 27.5 |
| | 6 | 12 | 18 | 28.5 |
| | 7 | 12 | 18 | 28.1 |
| | 9 | 12 | 18 | 28.0 |
| 18s rRNA | 21 | 31 | 40 | 24.9 |
| | 22 | 32 | 40 | 25.4 |
| PMS1 | 45 | 49 | 54 | 31.6 |
| | 46 | 50 | 54 | 32.0 |
| | 47 | 51 | 54 | 32.3 |
| | 48 | 52 | 54 | 32.2 |
| Mlh1a | 57 | 67 | 76 | 32.7 |
| | 58 | 68 | 76 | 33.4 |
| | 63 | 73 | 79 | 32.5 |
| | 64 | 74 | 79 | 32.9 |
| CRN | 80 | 90 | 100 | 34.6 |
| | 81 | 91 | 100 | 35.0 |
| | 82 | 92 | 101 | 33.5 |
| | 83 | 93 | 101 | 33.5 |

### EXAMPLE 2

The amplification and detection of the TV 5.8s rRNA gene was performed as described in Example 1 (using primers having SEQ ID NO:3 and 12 and a labeled probe having SEQ ID NO:18) with the exception that genomic template DNA for Mycoplasma genitalium (MG) was included in the PCR assay together with primers and probes that can amplify and detect MG. Herein, primers and probes that hybridize to the conserved region A of the mgpB gene (mgpB) having SEQ ID NOs: 139, 145, and 150 and to the variable region EF of the mgpB gene (MgPar) having SEQ ID NOs: 153, 169, and 194 were used.

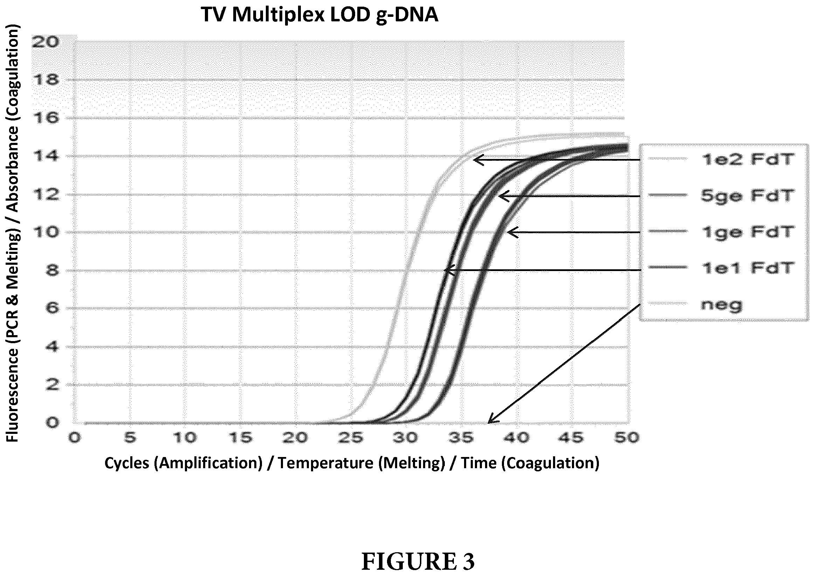

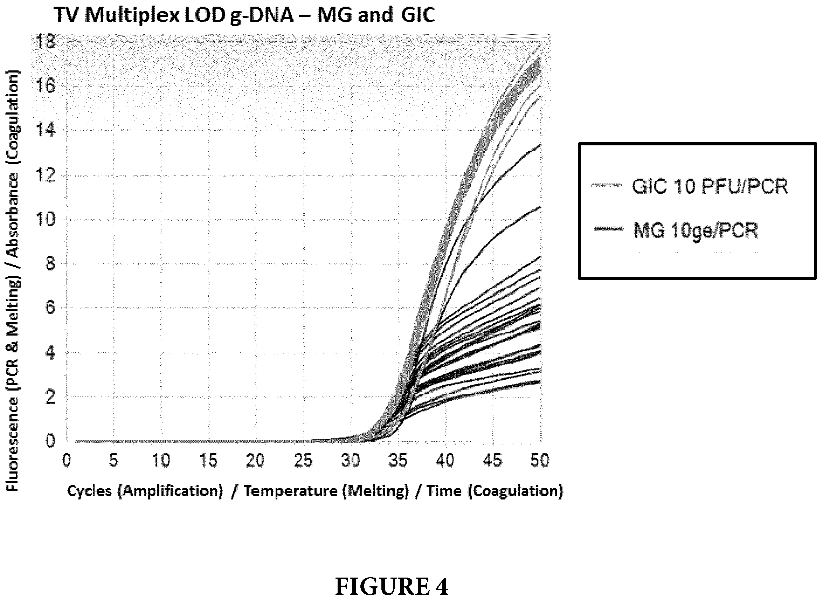

TV Limit of Detection (LOD) was tested at 100, 10, 5 and 1 genomic equivalent concentrations per PCR reaction (ge/PCR), in a co-amplification with internal control standard (GIC) and MG at 10 ge/PCR. The results are shown on Figure 3 (TV) and 4 (MG and GIC). All levels of TV were detected with no dropouts, and TV LOD is determined to be <1 ge/PCR. Further, MG at 10 ge/PCR could also be detected with no dropouts.

## Claims

1. A method of detecting *Trichomonas vaginalis* (TV) in a sample, the method comprising:
- performing an amplifying step comprising contacting the sample with a set of target TV gene primers to produce an amplification product if a target TV gene nucleic acid is present in the sample;
- performing a hybridizing step comprising contacting the amplification product with one or more detectable target TV gene probes; and
- detecting the presence or absence of the amplification product, wherein the presence of the amplification product is indicative of the presence of TV in the sample and wherein the absence of the amplification product is indicative of the absence of TV in the sample;
wherein the set of target TV gene primers comprise a first primer comprising a first oligonucleotide sequence selected from the group consisting of SEQ ID NOs: 1-9 or a complement thereof, and a second primer comprising a second oligonucleotide sequence selected from the group consisting of SEQ ID NOs: 10-13 or a complement thereof; and wherein the one or more detectable target TV gene probes comprises a third oligonucleotide sequence selected from the group consisting of SEQ ID NOs: 14-18 or the complement thereof.

2. The method of claim 1, wherein:
- the hybridizing step comprises contacting the amplification product with the detectable target TV gene probe that is labeled with a donor fluorescent moiety and a corresponding acceptor moiety; and
- the detecting step comprises detecting the presence or absence of fluorescence resonance energy transfer (FRET) between the donor fluorescent moiety and the acceptor moiety of the probe, wherein the presence or absence of fluorescence is indicative of the presence or absence of TV in the sample.

3. The method of any one of claims 1 to 2, wherein said amplifying step employs a polymerase enzyme having 5' to 3' nuclease activity.

4. The method of any one of claims 2 to 3, wherein the donor fluorescent moiety and the corresponding acceptor moiety are within no more than 8-20 nucleotides of each other on the probe.

5. The method of any one of claims 2 to 4, wherein the acceptor moiety is a quencher.

6. The method of any one of claims 1 to 5 further suitable for detecting *Mycoplasma genitalium* (MG) in said sample and further including:
- performing an amplifying step including contacting the sample with a set of primers designed to target a specific MG gene to produce an amplification product if MG is present in the sample;
- performing a hybridizing step including contacting the amplification product with one or more detectable probes to the target MG gene; and
- detecting the presence or absence of the amplified MG product,
wherein the presence of the amplified MG product is indicative of the presence of MG in the sample and wherein the absence of the amplified MG product is indicative of the absence of MG in the sample;
wherein the target MG gene is selected from the group consisting of the 23s ribosomal RNA (23s) gene, the conserved region A of the mgpB gene within the MgPa adhesion operon (mgpB), and the variable EF region of the mgpB partial repeats (MgPar).

7. The method of claim 6, wherein detecting TV and MG in the sample are performed in the same reaction mixture as a multiplex assay.

8. The method of any one of claims 7 and 8, wherein the set of target MG gene primers comprise a first primer comprising a first oligonucleotide sequence selected from the group consisting of SEQ ID NOs: 106-112, 134-140, and 152-161 or a complement thereof, and a second primer comprising a second oligonucleotide sequence selected from the group consisting of SEQ ID NOs: 113-122, 141-146, and 162-171, or a complement thereof; and wherein the one or more detectable target MG gene probes comprises a third oligonucleotide sequence selected from the group consisting of SEQ ID NOs: 123-133, 147-151, and 172-194, or the complement thereof.

9. A kit for detecting a nucleic acid of *Trichomonas vaginalis* (TV) comprising:
- a first primer comprising a first oligonucleotide sequence selected from the group consisting of SEQ ID NOs: 1-9 or a complement thereof;
- a second primer comprising a second oligonucleotide sequence selected from the group consisting of SEQ ID NOs: 10-13 or a complement thereof; and
- a fluorescently detectably labeled probe comprising a third oligonucleotide sequence selected from the group consisting of SEQ ID NOs: 14-18 or a complement thereof, the detectably labeled probe configured to hybridize to an amplicon generated by the first primer and the second primer.

10. The kit of claim 9, wherein the third detectably labeled oligonucleotide sequence comprises a donor fluorescent moiety and a corresponding acceptor moiety.

11. The kit of claim 10, wherein the acceptor moiety is a quencher.

12. The kit of any one of claims 9 to 11 further comprising at least one of nucleoside triphosphates, nucleic acid polymerase, and buffers necessary for the function of the nucleic acid polymerase.

13. The kit of any one of claims 9 to 12, wherein at least one of the first, second, and third oligonucleotides comprises at least one modified nucleotide.

14. The kit of any one of claims 9 to 13, wherein the first, second, and third oligonucleotides have 40 or fewer nucleotides.

15. The kit of any one of claims 9 to 14 further suitable for detecting a nucleic acid of *Mycoplasma genitalium* (MG) and further comprising a set of primers designed to target a specific MG gene to produce an amplification product of said MG gene and one or more detectable probes capable to hybridize to the MG amplification product.

## Patentansprüche

1. Verfahren zum Nachweisen von *Trichomonas vaginalis* (TV) in einer Probe, wobei das Verfahren Folgendes umfasst:
- Durchführen eines Amplifizierungsschrittes, umfassend das Inkontaktbringen der Probe mit einem Satz von Ziel-TV-Gen-Primern zum Produzieren eines Amplifikationsproduktes, wenn eine Ziel-TV-Gennukleinsäure in der Probe vorliegt;
- Durchführen eines Hybridisierungsschrittes, umfassend das Inkontaktbringen des Amplifikationsproduktes mit einer oder mehreren nachweisbaren Ziel-TV-Gensonden und
- Nachweisen der Gegenwart oder Abwesenheit des Amplifikationsproduktes, wobei die Gegenwart des Amplifikationsproduktes die Gegenwart von TV in der Probe angibt und wobei die Abwesenheit des Amplifikationsproduktes die Abwesenheit von TV in der Probe angibt;
wobei der Satz von Ziel-TV-Gen-Primern einen ersten Primer, umfassend eine erste Oligonukleotidsequenz, ausgewählt aus der Gruppe, bestehend aus SEQ ID NO: 1-9, oder ein Komplement davon, und einen zweiten Primer, umfassend eine zweite Oligonukleotidsequenz, ausgewählt aus der Gruppe, bestehend aus SEQ ID NO:10-13, oder ein Komplement davon, umfasst und wobei die eine oder mehreren nachweisbaren Ziel-TV-Gensonden eine dritte Oligonukleotidsequenz, ausgewählt aus der Gruppe, bestehend aus SEQ ID NO: 14-18, oder das Komplement davon umfasst/umfassen.

2. Verfahren nach Anspruch 1, wobei:
- der Hybridisierungsschritt das Inkontaktbringen des Amplifikationsproduktes mit der nachweisbaren Ziel-TV-Gensonde umfasst, die mit einer Donor-Fluoreszenzeinheit und einer entsprechenden Akzeptor-Einheit markiert ist; und
- der Nachweisschritt das Nachweisen der Gegenwart oder Abwesenheit von Fluoreszenz-Resonanzenergietransfer (FRET) zwischen der Donor-Fluoreszenzeinheit und der Akzeptor-Einheit der Sonde umfasst, wobei die Gegenwart oder Abwesenheit von Fluoreszenz die Gegenwart oder Abwesenheit von TV in der Probe angibt.

3. Verfahren nach einem der Ansprüche 1 bis 2, wobei der Amplifizierungsschritt ein Polymeraseenzym mit 5'- zu 3'-Nukleaseaktivität nutzt.

4. Verfahren nach einem der Ansprüche 2 bis 3, wobei sich die Donor-Fluoreszenzeinheit und die entsprechende Akzeptor-Einheit nicht mehr als 8 - 20 Nukleotide voneinander entfernt auf der Sonde befinden.

5. Verfahren nach einem der Ansprüche 2 bis 4, wobei die Akzeptor-Einheit ein Quencher ist.

6. Verfahren nach einem der Ansprüche 1 bis 5, das ferner zum Nachweisen von *Mycoplasma genitalium* (MG) in der Probe geeignet ist und ferner Folgendes einschließt:
- Durchführen eines Amplifizierungsschrittes, einschließend das Inkontaktbringen der Probe mit einem Satz von Primern, die dazu ausgelegt sind, ein spezifisches MG-Gen zu targetieren, um ein Amplifikationsprodukt zu produzieren, wenn MG in der Probe vorliegt;
- Durchführen eines Hybridisierungsschrittes, einschließend das Inkontaktbringen des Amplifikationsproduktes mit einer oder mehreren nachweisbaren Sonden für das Ziel-MG-Gen und
- Nachweisen der Gegenwart oder Abwesenheit des amplifizierten MG-Produktes, wobei die Gegenwart des amplifizierten MG-Produktes die Gegenwart von MG in der Probe angibt und wobei die Abwesenheit des amplifizierten MG-Produktes die Abwesenheit von MG in der Probe angibt;
wobei das Ziel-MG-Gen ausgewählt ist aus der Gruppe, bestehend aus dem 23s-ribosomale-RNA-(23s)-Gen, der konservierten Region A des mgpB-Gens innerhalb des MgPa-Adhäsionsoperons (mgpB) und der variablen EF-Region der mgpB-Teilwiederholungen (MgPar).

7. Verfahren nach Anspruch 6, wobei das Nachweisen von TV und MG in der Probe in demselben Reaktionsgemisch als ein Multiplex-Assay vorgenommen wird.

8. Verfahren nach einem der Ansprüche 7 und 8, wobei der Satz von Ziel-MG-Gen-Primern einen ersten Primer, umfassend eine erste Oligonukleotidsequenz, ausgewählt aus der Gruppe, bestehend aus SEQ ID NO:106-112, 134-140 und 152-161, oder ein Komplement davon, und einen zweiten Primer, umfassend eine zweite Oligonukleotidsequenz, ausgewählt aus der Gruppe, bestehend aus SEQ ID NO: 113-122, 141-146 und 162-171, oder ein Komplement davon, umfasst und wobei die eine oder mehreren nachweisbaren Ziel-MG-Gensonden eine dritte Oligonukleotidsequenz, ausgewählt aus der Gruppe, bestehend aus SEQ ID N0:123-133, 147-151 und 172-194, oder das Komplement davon umfasst/umfassen.

9. Kit zum Nachweisen einer Nukleinsäure von *Trichomonas vaginalis* (TV), umfassend:
- einen ersten Primer, umfassend eine erste Oligonukleotidsequenz, ausgewählt aus der Gruppe, bestehend aus SEQ ID NO:1-9, oder ein Komplement davon;
- einen zweiten Primer, umfassend eine zweite Oligonukleotidsequenz, ausgewählt aus der Gruppe, bestehend aus SEQ ID NO:10-13, oder ein Komplement davon; und
- eine fluoreszent nachweisbar markierte Sonde, umfassend eine dritte Oligonukleotidsequenz, ausgewählt aus der Gruppe, bestehend aus SEQ ID NO:14-18, oder ein Komplement davon, wobei die nachweisbar markierte Sonde dazu ausgelegt ist, an ein Amplicon zu hybridisieren, das von dem ersten Primer und dem zweiten Primer erzeugt wurde.

10. Kit nach Anspruch 9, wobei die dritte nachweisbar markierte Oligonukleotidsequenz eine Donor-Fluoreszenzeinheit und eine entsprechende Akzeptor-Einheit umfasst.

11. Kit nach Anspruch 10, wobei die Akzeptor-Einheit ein Quencher ist.

12. Kit nach einem der Ansprüche 9 bis 11, ferner umfassend mindestens eines von Nukleosidtriphosphaten, Nukleinsäurepolymerase und Puffern, die für die Funktion der Nukleinsäurepolymerase notwendig sind.

13. Kit nach einem der Ansprüche 9 bis 12, wobei mindestens eines von dem ersten, zweiten und dritten Oligonukleotid mindestens ein modifiziertes Nukleotid umfasst.

14. Kit nach einem der Ansprüche 9 bis 13, wobei das erste, zweite und dritte Oligonukleotid 40 oder weniger Nukleotide aufweisen.

15. Kit nach einem der Ansprüche 9 bis 14, der ferner zum Nachweisen einer Nukleinsäure von *Mycoplasma genitalium* (MG) geeignet ist und ferner einen Satz von Primern, die dazu ausgelegt sind, ein spezifisches MG-Gen zu targetieren, um ein Amplifikationsprodukt des MG-Gens zu produzieren, und eine oder mehrere nachweisbare Sonden, die an das MG-Amplifikationsprodukt hybridisieren können, umfasst.

## Revendications

1. Méthode de détection de *Trichomonas vaginalis* (TV) dans un échantillon, la méthode comprenant :
- la réalisation d'une étape d'amplification comprenant la mise en contact de l'échantillon avec un ensemble d'amorces de gène de TV cible pour produire un produit d'amplification si un acide nucléique de gène de TV cible est présent dans l'échantillon ;
- la réalisation d'une étape d'hybridation comprenant la mise en contact du produit d'amplification avec une ou plusieurs sondes de gène de TV cible détectables ; et
- la détection de la présence ou de l'absence du produit d'amplification, dans laquelle la présence du produit d'amplification indique la présence de TV dans l'échantillon et dans laquelle l'absence du produit d'amplification indique l'absence de TV dans l'échantillon ;
dans laquelle l'ensemble d'amorces de gène de TV cible comprend une première amorce comprenant une première séquence oligonucléotidique choisie dans le groupe constitué par les SEQ ID NO : 1 à 9 ou un complément de celles-ci, et une seconde amorce comprenant une deuxième séquence oligonucléotidique choisie dans le groupe constitué par les SEQ ID NO : 10 à 13 ou un complément de celles-ci ; et dans laquelle la ou les sondes de gène de TV cible détectables comprennent une troisième séquence oligonucléotidique choisie dans le groupe constitué par les SEQ ID NO : 14 à 18 ou le complément de celles-ci.

2. Méthode selon la revendication 1, dans laquelle :
- l'étape d'hybridation comprend la mise en contact du produit d'amplification avec la sonde de gène de TV cible détectable qui est marquée avec une fraction fluorescente donneuse et une fraction acceptrice correspondante ; et
- l'étape de détection comprend la détection de la présence ou de l'absence d'un transfert d'énergie par résonance de fluorescence (FRET) entre la fraction fluorescente donneuse et la fraction acceptrice de la sonde, dans laquelle la présence ou l'absence de fluorescence indique la présence ou l'absence de TV dans l'échantillon.

3. Méthode selon l'une quelconque des revendications 1 à 2, dans laquelle ladite étape d'amplification utilise une enzyme polymérase ayant une activité nucléase 5' vers 3'.

4. Méthode selon l'une quelconque des revendications 2 à 3, dans laquelle la fraction fluorescente donneuse et la fraction acceptrice correspondante sont espacées l'une de l'autre par pas plus de 8 à 20 nucléotides sur la sonde.

5. Méthode selon l'une quelconque des revendications 2 à 4, dans laquelle la fraction acceptrice est un extincteur.

6. Méthode selon l'une quelconque des revendications 1 à 5 en outre adaptée à la détection de *Mycoplasma genitalium* (MG) dans ledit échantillon et incluant en outre :
- la réalisation d'une étape d'amplification incluant la mise en contact de l'échantillon avec un ensemble d'amorces conçues pour cibler un gène de MG spécifique pour produire un produit d'amplification si MG est présent dans l'échantillon ;
- la réalisation d'une étape d'hybridation incluant la mise en contact du produit d'amplification avec une ou plusieurs sondes détectables pour le gène de MG cible ; et
- la détection de la présence ou de l'absence du produit de MG amplifié,
dans laquelle la présence du produit de MG amplifié indique la présence de MG dans l'échantillon et dans laquelle l'absence du produit de MG amplifié indique l'absence de MG dans l'échantillon ;
dans laquelle le gène de MG cible est choisi dans le groupe constitué par le gène de l'ARN ribosomique 23s (23s), la région conservée A du gène mgpB au sein de l'opéron d'adhésion MgPa (mgpB) et la région EF variable des répétitions partielles de mgpB (MgPar).

7. Méthode selon la revendication 6, dans laquelle la détection de TV et de MG dans l'échantillon est réalisée dans le même mélange réactionnel sous forme d'un dosage multiplex.

8. Méthode selon l'une quelconque des revendications 7 et 8, dans laquelle l'ensemble d'amorces de gène de MG cible comprend une première amorce comprenant une première séquence oligonucléotidique choisie dans le groupe constitué par les SEQ ID NO : 106 à 112, 134 à 140 et 152 à 161 ou un complément de celles-ci, et une seconde amorce comprenant une deuxième séquence oligonucléotidique choisie dans le groupe constitué par les SEQ ID NO : 113 à 122, 141 à 146 et 162 à 171 ou un complément de celles-ci ; et dans laquelle la ou les sondes de gène de MG cible détectables comprennent une troisième séquence oligonucléotidique choisie dans le groupe constitué par les SEQ ID NO : 123 à 133, 147 à 151 et 172 à 194 ou le complément de celles-ci.

9. Kit de détection d'un acide nucléique de *Trichomonas vaginalis* (TV) comprenant :
- une première amorce comprenant une première séquence oligonucléotidique choisie dans le groupe constitué par les SEQ ID NO : 1 à 9 ou un complément de celles-ci ;
- une seconde amorce comprenant une deuxième séquence oligonucléotidique choisie dans le groupe constitué par les SEQ ID NO : 10 à 13 ou un complément de celles-ci ; et
- une sonde marquée de manière détectable par fluorescence comprenant une troisième séquence oligonucléotidique choisie dans le groupe constitué par les SEQ ID NO : 14 à 18 ou un complément de celles-ci, la sonde marquée de manière détectable étant configurée pour s'hybrider à un amplicon généré par la première amorce et la seconde amorce.

10. Kit selon la revendication 9, dans lequel la troisième séquence oligonucléotidique marquée de manière détectable comprend une fraction fluorescente donneuse et une fraction acceptrice correspondante.

11. Kit selon la revendication 10, dans lequel la fraction acceptrice est un extincteur.

12. Kit selon l'une quelconque des revendications 9 à 11 comprenant en outre au moins un des triphosphates de nucléoside, d'une acide nucléique polymérase et des tampons nécessaires pour la fonction de l'acide nucléique polymérase.

13. Kit selon l'une quelconque des revendications 9 à 12, dans lequel au moins un des premier, deuxième et troisième oligonucléotides comprend au moins un nucléotide modifié.

14. Kit selon l'une quelconque des revendications 9 à 13, dans lequel les premier, deuxième et troisième oligonucléotides ont 40 nucléotides ou moins.

15. Kit selon l'une quelconque des revendications 9 à 14 en outre adapté à la détection d'un acide nucléique de *Mycoplasma genitalium* (MG) et comprenant en outre un ensemble d'amorces conçues pour cibler un gène de MG spécifique pour produire un produit d'amplification dudit gène de MG et une ou plusieurs sondes détectables capables de s'hybrider au produit d'amplification de MG.


### SEQUENCE LISTING

<110> Roche Diagnostics GmbH
   F. Hoffmann-La Roche AG
   Roche Molecular Systems, Inc.
<120> COMPOSITIONS AND METHODS FOR DETECTION OF TRICHOMONAS VAGINALIS
<130> P33593-WO-HS
<150> US 62/342600
   <151> 2016-05-27
<150> US 62/342519
   <151> 2016-05-27
<160> 194
<170> PatentIn version 3.5
<210> 1
   <211> 24
   <212> DNA
   <213> Artificial Sequence
<220>
   <223> 5.8s forward primer
<220>
   <221> modified_base
   <222> (24) .. (24)
   <223> t-butylbenzyldC
<400> 1
   ccaagtctct aagcaatgga tgtc 24
<210> 2
   <211> 21
   <212> DNA
   <213> Artificial Sequence
<220>
   <223> 5.8s forward primer
<220>
   <221> modified_base
   <222> (21)..(21)
   <223> t-butylbenzyldC
<400> 2
   aagcaatgga tgtcttggct c 21
<210> 3
   <211> 24
   <212> DNA
   <213> Artificial Sequence
<220>
   <223> 5.8s forward primer
<220>
   <221> modified_base
   <222> (24)..(24)
   <223> t-butylbenzyldC
<400> 3
   tgttaagtaa ccggagttgc aaac 24
<210> 4
   <211> 22
   <212> DNA
   <213> Artificial Sequence
<220>
   <223> 5.8s forward primer
<220>
   <221> modified_base
   <222> (22)..(22)
   <223> t-butylbenzyldC
<400> 4
   ttaagtaacc ggagttgcaa ac 22
<210> 5
   <211> 23
   <212> DNA
   <213> Artificial Sequence
<220>
   <223> 5.8s forward primer
<220>
   <221> modified_base
   <222> (23)..(23)
   <223> t-butylbenzyldC
<400> 5
   caaattgcgc taaactcgat ctc 23
<210> 6
   <211> 20
   <212> DNA
   <213> Artificial Sequence
<220>
   <223> 5.8s forward primer
<220>
   <221> modified_base
   <222> (20)..(20)
   <223> t-butylbenzyldA
<400> 6
   ctaaactcga tctcggtcga 20
<210> 7
   <211> 20
   <212> DNA
   <213> Artificial Sequence
<220>
   <223> 5.8s forward primer
<220>
   <221> modified_base
   <222> (20)..(20)
   <223> t-butylbenzyldC
<400> 7
   cgctaaactc gatctcggtc 20
<210> 8
   <211> 22
   <212> DNA
   <213> Artificial Sequence
<220>
   <223> 5.8s forward primer
<220>
   <221> modified_base
   <222> (22)..(22)
   <223> t-butylbenzyldC
<400> 8
   aaattgcgct aaactcgatc tc 22
<210> 9
   <211> 22
   <212> DNA
   <213> Artificial Sequence
<220>
   <223> 5.8s forward primer
<220>
   <221> modified_base
   <222> (22)..(22)
   <223> t-butylbenzyldC
<400> 9
   gcaaattgcg ctaaactcga tc 22
<210> 10
   <211> 19
   <212> DNA
   <213> Artificial Sequence
<220>
   <223> 5.8s reverse primer
<220>
   <221> modified_base
   <222> (19)..(19)
   <223> t-butylbenzyldA
<400> 10
   tcacacccat gcttctcga 19
<210> 11
   <211> 20
   <212> DNA
   <213> Artificial Sequence
<220>
   <223> 5.8s reverse primer
<220>
   <221> modified_base
   <222> (20)..(20)
   <223> t-butylbenzyldC
<400> 11
   catgcttctc gaccgagatc 20
<210> 12
   <211> 37
   <212> DNA
   <213> Artificial Sequence
<220>
   <223> 5.8s reverse primer
<220>
   <221> modified_base
   <222> (37)..(37)
   <223> t-butylbenzyldA
<400> 12
   tgtttgtctt atatattatt tacttattcg cttagaa 37
<210> 13
   <211> 35
   <212> DNA
   <213> Artificial Sequence
<220>
   <223> 5.8s reverse primer
<220>
   <221> modified_base
   <222> (35)..(35)
   <223> t-butylbenzyldA
<400> 13
   tttgtcttat atattattta cttattcgct tagaa 35
<210> 14
   <211> 35
   <212> DNA
   <213> Artificial Sequence
<220>
   <223> 5.8s probe
<220>
   <221> misc_feature
   <223> 5' FAM
<220>
   <221> misc_feature
   <223> 3' phosphate
<220>
   <221> misc_feature
   <223> 3' phosphate
<220>
   <221> misc_feature
   <222> (6)..(7)
   <223> BHQ-2
<400> 14
   aacatcatga caggttaatc tttgaatgca aattg 35
<210> 15
   <211> 28
   <212> DNA
   <213> Artificial Sequence
<220>
   <223> 5.8s probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 15
   taaccggagt tgcaaacatc atgacagg 28
<210> 16
   <211> 26
   <212> DNA
   <213> Artificial Sequence
<220>
   <223> 5.8s probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 16
   attgcgctaa actcgatctc ggtcga 26
<210> 17
   <211> 29
   <212> DNA
   <213> Artificial Sequence
<220>
   <223> 5.8s probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 17
   ctaaactcga tctcggtcga gaagcatgg 29
<210> 18
   <211> 32
   <212> DNA
   <213> Artificial Sequence
<220>
   <223> 5.8s probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 18
   tcgagaagca tgggtgtgac agtactacat ct 32
<210> 19
   <211> 27
   <212> DNA
   <213> Artificial Sequence
<220>
   <223> 18s forward primer
<220>
   <221> modified_base
   <222> (27)..(27)
   <223> t-benzyldA
<400> 19
   cgtagttggg attgacgttt gtaatca 27
<210> 20
   <211> 27
   <212> DNA
   <213> Artificial Sequence
<220>
   <223> 18s forward primer
<220>
   <221> modified_base
   <222> (27)..(27)
   <223> t-butylbenzyldA
<400> 20
   cgtagttggg attgacgttt gtaatca 27
<210> 21
   <211> 21
   <212> DNA
   <213> Artificial Sequence
<220>
   <223> 18s forward primer
<220>
   <221> modified_base
   <222> (21)..(21)
   <223> t-benzyldA
<400> 21
   gggaaactta ccaggaccag a 21
<210> 22
   <211> 21
   <212> DNA
   <213> Artificial Sequence
<220>
   <223> 18s forward primer
<220>
   <221> modified_base
   <222> (21)..(21)
   <223> t-butylbenzyldA
<400> 22
   gggaaactta ccaggaccag a 21
<210> 23
   <211> 28
   <212> DNA
   <213> Artificial Sequence
<220>
   <223> 18s forward primer
<220>
   <221> modified_base
   <222> (28)..(28)
   <223> t-benzyldA
<400> 23
   gaaacttacc aggaccagat gtttttta 28
<210> 24
   <211> 28
   <212> DNA
   <213> Artificial Sequence
<220>
   <223> 18s forward primer
<220>
   <221> modified_base
   <222> (28)..(28)
   <223> t-butylbenzyldA
<400> 24
   gaaacttacc aggaccagat gtttttta 28
<210> 25
   <211> 26
   <212> DNA
   <213> Artificial Sequence
<220>
   <223> 18s forward primer
<220>
   <221> modified_base
   <222> (26)..(26)
   <223> t-benzyldA
<400> 25
   cttgaaggaa ttgacggaag ggcaca 26
<210> 26
   <211> 26
   <212> DNA
   <213> Artificial Sequence
<220>
   <223> 18s forward primer
<220>
   <221> modified_base
   <222> (26)..(26)
   <223> t-butylbenzyldA
<400> 26
   cttgaaggaa ttgacggaag ggcaca 26
<210> 27
   <211> 24
   <212> DNA
   <213> Artificial Sequence
<220>
   <223> 18s forward primer
<220>
   <221> modified_base
   <222> (24)..(24)
   <223> t-benzyldA
<400> 27
   gccattcgac tgagtgacct atca 24
<210> 28
   <211> 24
   <212> DNA
   <213> Artificial Sequence
<220>
   <223> 18s forward primer
<220>
   <221> modified_base
   <222> (24)..(24)
   <223> t-butylbenzyldA
<400> 28
   gccattcgac tgagtgacct atca 24
<210> 29
   <211> 27
   <212> DNA
   <213> Artificial Sequence
<220>
   <223> 18s reverse primer
<220>
   <221> modified_base
   <222> (27)..(27)
   <223> t-benzyldA
<400> 29
   gacttctcct tcctctagat aacgtga 27
<210> 30
   <211> 27
   <212> DNA
   <213> Artificial Sequence
<220>
   <223> 18s reverse primer
<220>
   <221> modified_base
   <222> (27)..(27)
   <223> t-butylbenzyldA
<400> 30
   gacttctcct tcctctagat aacgtga 27
<210> 31
   <211> 25
   <212> DNA
   <213> Artificial Sequence
<220>
   <223> 18s reverse primer
<220>
   <221> modified_base
   <222> (25)..(25)
   <223> t-benzyldA
<400> 31
   ttgctaccct cttccacctg ctaaa 25
<210> 32
   <211> 25
   <212> DNA
   <213> Artificial Sequence
<220>
   <223> 18s reverse primer
<220>
   <221> modified_base
   <222> (25)..(25)
   <223> t-butylbenzyldA
<400> 32
   ttgctaccct cttccacctg ctaaa 25
<210> 33
   <211> 26
   <212> DNA
   <213> Artificial Sequence
<220>
   <223> 18s reverse primer
<220>
   <221> modified_base
   <222> (26)..(26)
   <223> t-benzyldC
<400> 33
   gctaccctct tccacctgct aaaatc 26
<210> 34
   <211> 26
   <212> DNA
   <213> Artificial Sequence
<220>
   <223> 18s reverse primer
<220>
   <221> modified_base
   <222> (26)..(26)
   <223> t-butylbenzyldC
<400> 34
   gctaccctct tccacctgct aaaatc 26
<210> 35
   <211> 24
   <212> DNA
   <213> Artificial Sequence
<220>
   <223> 18s reverse primer
<220>
   <221> modified_base
   <222> (24)..(24)
   <223> t-benzyldC
<400> 35
   tgaatcaacg ctagacaggt caac 24
<210> 36
   <211> 24
   <212> DNA
   <213> Artificial Sequence
<220>
   <223> 18s reverse primer
<220>
   <221> modified_base
   <222> (24)..(24)
   <223> t-butylbenzyldC
<400> 36
   tgaatcaacg ctagacaggt caac 24
<210> 37
   <211> 27
   <212> DNA
   <213> Artificial Sequence
<220>
   <223> 18s reverse primer
<220>
   <221> modified_base
   <222> (27)..(27)
   <223> t-benzyldA
<400> 37
   aaaaggcacc aatggaactg gtcatta 27
<210> 38
   <211> 27
   <212> DNA
   <213> Artificial Sequence
<220>
   <223> 18s reverse primer
<220>
   <221> modified_base
   <222> (27)..(27)
   <223> t-butylbenzyldA
<400> 38
   aaaaggcacc aatggaactg gtcatta 27
<210> 39
   <211> 30
   <212> DNA
   <213> Artificial Sequence
<220>
   <223> 18s probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 39
   aatcccttgt aaatgtgtgt caacaacgca 30
<210> 40
   <211> 32
   <212> DNA
   <213> Artificial Sequence
<220>
   <223> 18s probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 40
   ccaccaaaaa caatatcctg aaagacccga ag 32
<210> 41
   <211> 32
   <212> DNA
   <213> Artificial Sequence
<220>
   <223> 18s probe
<220>
   <221> misc_feature
   <223> 5' FAM
<220>
   <221> misc_feature
   <223> 3' phosphate
<220>
   <221> misc_feature
   <222> (6).. (7)
   <223> BHQ-2
<400> 41
   ccaaaaacaa tatcctgaaa gacccgaagc ct 32
<210> 42
   <211> 32
   <212> DNA
   <213> Artificial Sequence
<220>
   <223> 18s probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 42
   accaaaaaca atatcctgaa agacccgaag cc 32
<210> 43
   <211> 31
   <212> DNA
   <213> Artificial Sequence
<220>
   <223> 18s probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 43
   ctgctacccg tggatatagt cgctatctct c 31
<210> 44
   <211> 31
   <212> DNA
   <213> Artificial Sequence
<220>
   <223> 18s probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 44
   ctgagagata gcgactatat ccacgggtag c 31
<210> 45
   <211> 27
   <212> DNA
   <213> Artificial Sequence
<220>
   <223> PMS1 forward primer
<220>
   <221> modified_base
   <222> (27)..(27)
   <223> t-benzyldA
<400> 45
   ccgagagatg attgagaacg tatttga 27
<210> 46
   <211> 27
   <212> DNA
   <213> Artificial Sequence
<220>
   <223> PMS1 forward primer
<220>
   <221> modified_base
   <222> (27)..(27)
   <223> t-butylbenzyldA
<400> 46
   ccgagagatg attgagaacg tatttga 27
<210> 47
   <211> 26
   <212> DNA
   <213> Artificial Sequence
<220>
   <223> PMS1 forward primer
<220>
   <221> modified_base
   <222> (26).. (26)
   <223> t-benzyldA
<400> 47
   cactccgaga gatgattgag aacgta 26
<210> 48
   <211> 26
   <212> DNA
   <213> Artificial Sequence
<220>
   <223> PMS1 forward primer
<220>
   <221> modified_base
   <222> (26)..(26)
   <223> t-butylbenzyldA
<400> 48
   cactccgaga gatgattgag aacgta 26
<210> 49
   <211> 24
   <212> DNA
   <213> Artificial Sequence
<220>
   <223> PMS1 reverse primer
<220>
   <221> modified_base
   <222> (24)..(24)
   <223> t-benzyldC
<400> 49
   gccacttaca tcttttccaa attc 24
<210> 50
   <211> 24
   <212> DNA
   <213> Artificial Sequence
<220>
   <223> PMS1 reverse primer
<220>
   <221> modified_base
   <222> (24)..(24)
   <223> t-butylbenzyldC
<400> 50
   gccacttaca tcttttccaa attc 24
<210> 51
   <211> 28
   <212> DNA
   <213> Artificial Sequence
<220>
   <223> PMS1 reverse primer
<220>
   <221> modified_base
   <222> (28)..(28)
   <223> t-benzyldA
<400> 51
   gtgacacctt catcacaaat cattgaaa 28
<210> 52
   <211> 28
   <212> DNA
   <213> Artificial Sequence
<220>
   <223> PMS1 reverse primer
<220>
   <221> modified_base
   <222> (28)..(28)
   <223> t-butylbenzyldA
<400> 52
   gtgacacctt catcacaaat cattgaaa 28
<210> 53
   <211> 30
   <212> DNA
   <213> Artificial Sequence
<220>
   <223> PMS1 probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 53
   ccaccatttc caactcgaat tgtcaaaagt 30
<210> 54
   <211> 32
   <212> DNA
   <213> Artificial Sequence
<220>
   <223> PMS1 probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 54
   ccaccatttc caactcgaat tgtcaaaagt gt 32
<210> 55
   <211> 25
   <212> DNA
   <213> Artificial Sequence
<220>
   <223> Mlh 1a forward primer
<220>
   <221> modified_base
   <222> (25)..(25)
   <223> t-benzyldA
<400> 55
   ctcctgtatc tataaatgaa gagaa 25
<210> 56
   <211> 25
   <212> DNA
   <213> Artificial Sequence
<220>
   <223> Mlh 1a forward primer
<220>
   <221> modified_base
   <222> (25)..(25)
   <223> t-butylbenzyldA
<400> 56
   ctcctgtatc tataaatgaa gagaa 25
<210> 57
   <211> 27
   <212> DNA
   <213> Artificial Sequence
<220>
   <223> Mlh 1a forward primer
<220>
   <221> modified_base
   <222> (27)..(27)
   <223> t-benzyldA
<400> 57
   gatttctgat aatggctgtg gaataaa 27
<210> 58
   <211> 27
   <212> DNA
   <213> Artificial Sequence
<220>
   <223> Mlh 1a forward primer
<220>
   <221> modified_base
   <222> (27)..(27)
   <223> t-butylbenzyldA
<400> 58
   gatttctgat aatggctgtg gaataaa 27
<210> 59
   <211> 27
   <212> DNA
   <213> Artificial Sequence
<220>
   <223> Mlh 1a forward primer
<220>
   <221> modified_base
   <222> (27)..(27)
   <223> t-benzyldA
<400> 59
   gaattatctc ctgtatctat aaatgaa 27
<210> 60
   <211> 27
   <212> DNA
   <213> Artificial Sequence
<220>
   <223> Mlh 1a forward primer
<220>
   <221> modified_base
   <222> (27)..(27)
   <223> t-butylbenzyldA
<400> 60
   gaattatctc ctgtatctat aaatgaa 27
<210> 61
   <211> 24
   <212> DNA
   <213> Artificial Sequence
<220>
   <223> Mlh 1a forward primer
<220>
   <221> modified_base
   <222> (24)..(24)
   <223> t-benzyldC
<400> 61
   agtaacagca agttcacttt tgtc 24
<210> 62
   <211> 24
   <212> DNA
   <213> Artificial Sequence
<220>
   <223> Mlh 1a forward primer
<220>
   <221> modified_base
   <222> (24)..(24)
   <223> t-butylbenzyldC
<400> 62
   agtaacagca agttcacttt tgtc 24
<210> 63
   <211> 23
   <212> DNA
   <213> Artificial Sequence
<220>
   <223> Mlh 1a forward primer
<220>
   <221> modified_base
   <222> (23)..(23)
   <223> t-benzyldA
<400> 63
   caggtgatat cgcgaagaac aca 23
<210> 64
   <211> 23
   <212> DNA
   <213> Artificial Sequence
<220>
   <223> Mlh 1a forward primer
<220>
   <221> modified_base
   <222> (23)..(23)
   <223> t-butylbenzyldA
<400> 64
   caggtgatat cgcgaagaac aca 23
<210> 65
   <211> 26
   <212> DNA
   <213> Artificial Sequence
<220>
   <223> Mlh 1a reverse primer
<220>
   <221> modified_base
   <222> (26)..(26)
   <223> t-benzyldA
<400> 65
   ggaatatttg attttggaat ttcaga 26
<210> 66
   <211> 26
   <212> DNA
   <213> Artificial Sequence
<220>
   <223> Mlh 1a reverse primer
<220>
   <221> modified_base
   <222> (26)..(26)
   <223> t-butylbenzyldA
<400> 66
   ggaatatttg attttggaat ttcaga 26
<210> 67
   <211> 26
   <212> DNA
   <213> Artificial Sequence
<220>
   <223> Mlh 1a reverse primer
<220>
   <221> modified_base
   <222> (26).. (26)
   <223> t-benzyldA
<400> 67
   ccaaatgaac tttcttctgt tttaga 26
<210> 68
   <211> 26
   <212> DNA
   <213> Artificial Sequence
<220>
   <223> Mlh 1a reverse primer
<220>
   <221> modified_base
   <222> (26)..(26)
   <223> t-butylbenzyldA
<400> 68
   ccaaatgaac tttcttctgt tttaga 26
<210> 69
   <211> 27
   <212> DNA
   <213> Artificial Sequence
<220>
   <223> Mlh 1a reverse primer
<220>
   <221> modified_base
   <222> (27)..(27)
   <223> t-benzyldC
<400> 69
   atttgatttt ggaatttcag agttttc 27
<210> 70
   <211> 27
   <212> DNA
   <213> Artificial Sequence
<220>
   <223> Mlh 1a reverse primer
<220>
   <221> modified_base
   <222> (27)..(27)
   <223> t-butylbenzyldC
<400> 70
   atttgatttt ggaatttcag agttttc 27
<210> 71
   <211> 26
   <212> DNA
   <213> Artificial Sequence
<220>
   <223> Mlh 1a reverse primer
<220>
   <221> modified_base
   <222> (26)..(26)
   <223> t-benzyldC
<400> 71
   ctgaagactt ggaatagatg tactgc 26
<210> 72
   <211> 26
   <212> DNA
   <213> Artificial Sequence
<220>
   <223> Mlh 1a reverse primer
<220>
   <221> modified_base
   <222> (26)..(26)
   <223> t-butylbenzyldC
<400> 72
   ctgaagactt ggaatagatg tactgc 26
<210> 73
   <211> 26
   <212> DNA
   <213> Artificial Sequence
<220>
   <223> Mlh 1a reverse primer
<220>
   <221> modified_base
   <222> (26)..(26)
   <223> t-benzyldA
<400> 73
   ggcatcctta ataaaacaaa agcaaa 26
<210> 74
   <211> 26
   <212> DNA
   <213> Artificial Sequence
<220>
   <223> Mlh 1a reverse primer
<220>
   <221> modified_base
   <222> (26).. (26)
   <223> t-butylbenzyldA
<400> 74
   ggcatcctta ataaaacaaa agcaaa 26
<210> 75
   <211> 32
   <212> DNA
   <213> Artificial Sequence
<220>
   <223> Mlh 1a probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 75
   aaaatccaag aaaaagagca agaagaaatc ct 32
<210> 76
   <211> 32
   <212> DNA
   <213> Artificial Sequence
<220>
   <223> Mlh 1a probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 76
   cacctctgaa tccaaatgta gttacgttcc tt 32
<210> 77
   <211> 32
   <212> DNA
   <213> Artificial Sequence
<220>
   <223> Mlh 1a probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 77
   cttgctcttt ttcttggatt ttctgtatct ga 32
<210> 78
   <211> 32
   <212> DNA
   <213> Artificial Sequence
<220>
   <223> Mlh 1a probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 78
   ttcaaaccaa tcaaaccaac aaaagaatga gc 32
<210> 79
   <211> 32
   <212> DNA
   <213> Artificial Sequence
<220>
   <223> Mlh 1a probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 79
   aaaaccgctg attctttgag ttgttttttg gc 32
<210> 80
   <211> 25
   <212> DNA
   <213> Artificial Sequence
<220>
   <223> CRN forward primer
<220>
   <221> modified_base
   <222> (25)..(25)
   <223> t-benzyldA
<400> 80
   gcaatctggg atctcaacaa ggaaa 25
<210> 81
   <211> 25
   <212> DNA
   <213> Artificial Sequence
<220>
   <223> CRN forward primer
<220>
   <221> modified_base
   <222> (25)..(25)
   <223> t-butylbenzyldA
<400> 81
   gcaatctggg atctcaacaa ggaaa 25
<210> 82
   <211> 21
   <212> DNA
   <213> Artificial Sequence
<220>
   <223> CRN forward primer
<220>
   <221> modified_base
   <222> (21)..(21)
   <223> t-benzyldC
<400> 82
   tttcatcgga cagggcaatc c 21
<210> 83
   <211> 21
   <212> DNA
   <213> Artificial Sequence
<220>
   <223> CRN forward primer
<220>
   <221> modified_base
   <222> (21)..(21)
   <223> t-butylbenzyldC
<400> 83
   tttcatcgga cagggcaatc c 21
<210> 84
   <211> 26
   <212> DNA
   <213> Artificial Sequence
<220>
   <223> CRN forward primer
<220>
   <221> modified_base
   <222> (26)..(26)
   <223> t-benzyldA
<400> 84
   gagggaccac aagaagaagt cgttca 26
<210> 85
   <211> 26
   <212> DNA
   <213> Artificial Sequence
<220>
   <223> CRN forward primer
<220>
   <221> modified_base
   <222> (26)..(26)
   <223> t-butylbenzyldA
<400> 85
   gagggaccac aagaagaagt cgttca 26
<210> 86
   <211> 24
   <212> DNA
   <213> Artificial Sequence
<220>
   <223> CRN forward primer
<220>
   <221> modified_base
   <222> (24)..(24)
   <223> t-benzyldC
<400> 86
   gacgagggac cacaagaaga agtc 24
<210> 87
   <211> 24
   <212> DNA
   <213> Artificial Sequence
<220>
   <223> CRN forward primer
<220>
   <221> modified_base
   <222> (24)..(24)
   <223> t-butylbenzyldC
<400> 87
   gacgagggac cacaagaaga agtc 24
<210> 88
   <211> 21
   <212> DNA
   <213> Artificial Sequence
<220>
   <223> CRN forward primer
<220>
   <221> modified_base
   <222> (21)..(21)
   <223> t-benzyldC
<400> 88
   cagagatcat ccagccagat c 21
<210> 89
   <211> 21
   <212> DNA
   <213> Artificial Sequence
<220>
   <223> CRN forward primer
<220>
   <221> modified_base
   <222> (21)..(21)
   <223> t-butylbenzyldC
<400> 89
   cagagatcat ccagccagat c 21
<210> 90
   <211> 24
   <212> DNA
   <213> Artificial Sequence
<220>
   <223> CRN reverse primer
<220>
   <221> modified_base
   <222> (24)..(24)
   <223> t-benzyldA
<400> 90
   ggttgtaatc tggaaggtcg agaa 24
<210> 91
   <211> 24
   <212> DNA
   <213> Artificial Sequence
<220>
   <223> CRN reverse primer
<220>
   <221> modified_base
   <222> (24)..(24)
   <223> t-butylbenzyldA
<400> 91
   ggttgtaatc tggaaggtcg agaa 24
<210> 92
   <211> 23
   <212> DNA
   <213> Artificial Sequence
<220>
   <223> CRN reverse primer
<220>
   <221> modified_base
   <222> (23)..(23)
   <223> t-benzyldC
<400> 92
   aacgtcagga acatcccaaa ggc 23
<210> 93
   <211> 23
   <212> DNA
   <213> Artificial Sequence
<220>
   <223> CRN reverse primer
<220>
   <221> modified_base
   <222> (23)..(23)
   <223> t-butylbenzyldC
<400> 93
   aacgtcagga acatcccaaa ggc 23
<210> 94
   <211> 27
   <212> DNA
   <213> Artificial Sequence
<220>
   <223> CRN reverse primer
<220>
   <221> modified_base
   <222> (27)..(27)
   <223> t-benzyldA
<400> 94
   gcgagttggc ttatcaaggt tcatgaa 27
<210> 95
   <211> 27
   <212> DNA
   <213> Artificial Sequence
<220>
   <223> CRN reverse primer
<220>
   <221> modified_base
   <222> (27)..(27)
   <223> t-butylbenzyldA
<400> 95
   gcgagttggc ttatcaaggt tcatgaa 27
<210> 96
   <211> 22
   <212> DNA
   <213> Artificial Sequence
<220>
   <223> CRN reverse primer
<220>
   <221> modified_base
   <222> (22)..(22)
   <223> t-benzyldC
<400> 96
   gttgattgga tagcgagttg gc 22
<210> 97
   <211> 22
   <212> DNA
   <213> Artificial Sequence
<220>
   <223> CRN reverse primer
<220>
   <221> modified_base
   <222> (22)..(22)
   <223> t-butylbenzyldC
<400> 97
   gttgattgga tagcgagttg gc 22
<210> 98
   <211> 21
   <212> DNA
   <213> Artificial Sequence
<220>
   <223> CRN reverse primer
<220>
   <221> modified_base
   <222> (21)..(21)
   <223> t-benzyldC
<400> 98
   ctcgtcgaca acttcctcct c 21
<210> 99
   <211> 21
   <212> DNA
   <213> Artificial Sequence
<220>
   <223> CRN reverse primer
<220>
   <221> modified_base
   <222> (21)..(21)
   <223> t-butylbenzyldC
<400> 99
   ctcgtcgaca acttcctcct c 21
<210> 100
   <211> 32
   <212> DNA
   <213> Artificial Sequence
<220>
   <223> CRN probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 100
   ccaagactca acattcaact cattatctaa cg 32
<210> 101
   <211> 31
   <212> DNA
   <213> Artificial Sequence
<220>
   <223> CRN probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 101
   acatcacata ctcaccacat aatccaaatc t 31
<210> 102
   <211> 31
   <212> DNA
   <213> Artificial Sequence
<220>
   <223> CRN probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 102
   acaatgccaa ctggaagata agtaaagtag t 31
<210> 103
   <211> 32
   <212> DNA
   <213> Artificial Sequence
<220>
   <223> CRN probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 103
   acaatgccaa ctggaagata agtaaagtag tt 32
<210> 104
   <211> 32
   <212> DNA
   <213> Artificial Sequence
<220>
   <223> CRN probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 104
   ttggaagatg ggatttgtca actgtcaatc tg 32
<210> 105
   <211> 31
   <212> DNA
   <213> Artificial Sequence
<220>
   <223> CRN probe
<220>
   <221> misc_feature
   <223> 5' FAM
<220>
   <221> misc_feature
   <223> 3' phosphate
<220>
   <221> misc_feature
   <222> (6)..(7)
   <223> BHQ-2
<400> 105
   ttggaagatg ggatttgtca actgtcaatc t 31
<210> 106
   <211> 21
   <212> DNA
   <213> Artificial Sequence
<220>
   <223> 23s forward primer
<220>
   <221> modified_base
   <222> (21)..(21)
   <223> t-butylbenzyldA
<400> 106
   ggggtggatc acctcctttc a 21
<210> 107
   <211> 25
   <212> DNA
   <213> Artificial Sequence
<220>
   <223> 23s forward primer
<220>
   <221> modified_base
   <222> (25)..(25)
   <223> t-butylbenzyldC
<400> 107
   caatgtttgg tctcacaact aacac 25
<210> 108
   <211> 26
   <212> DNA
   <213> Artificial Sequence
<220>
   <223> 23s forward primer
<220>
   <221> modified_base
   <222> (26)..(26)
   <223> t-butylbenzyldA
<400> 108
   tccagttctg aaagaatgtt tttgaa 26
<210> 109
   <211> 26
   <212> DNA
   <213> Artificial Sequence
<220>
   <223> 23s forward primer
<220>
   <221> modified_base
   <222> (26)..(26)
   <223> t-butylbenzyldA
<400> 109
   aaacgacaat ctttctagtt ccaaaa 26
<210> 110
   <211> 23
   <212> DNA
   <213> Artificial Sequence
<220>
   <223> 23s forward primer
<220>
   <221> modified_base
   <222> (23)..(23)
   <223> t-butylbenzyldC
<400> 110
   atgtttggtc tcacaactaa cac 23
<210> 111
   <211> 24
   <212> DNA
   <213> Artificial Sequence
<220>
   <223> 23s forward primer
<220>
   <221> modified_base
   <222> (24)..(24)
   <223> t-butylbenzyldA
<400> 111
   cagttctgaa agaatgtttt tgaa 24
<210> 112
   <211> 23
   <212> DNA
   <213> Artificial Sequence
<220>
   <223> 23s forward primer
<220>
   <221> modified_base
   <222> (23)..(23)
   <223> t-butylbenzyldA
<400> 112
   cgacaatctt tctagttcca aaa 23
<210> 113
   <211> 24
   <212> DNA
   <213> Artificial Sequence
<220>
   <223> 23s reverse primer
<220>
   <221> modified_base
   <222> (24)..(24)
   <223> t-butylbenzyldC
<400> 113
   cggatctcag gtttttacca cctc 24
<210> 114
   <211> 20
   <212> DNA
   <213> Artificial Sequence
<220>
   <223> 23s reverse primer
<220>
   <221> modified_base
   <222> (20)..(20)
   <223> t-butylbenzyldA
<400> 114
   cagattgctc cattcggaca 20
<210> 115
   <211> 21
   <212> DNA
   <213> Artificial Sequence
<220>
   <223> 23s reverse primer
<220>
   <221> modified_base
   <222> (21)..(21)
   <223> t-butylbenzyldC
<400> 115
   cagattgctc cattcggaca c 21
<210> 116
   <211> 20
   <212> DNA
   <213> Artificial Sequence
<220>
   <223> 23s reverse primer
<220>
   <221> modified_base
   <222> (20)..(20)
   <223> t-butylbenzyldC
<400> 116
   agattgctcc attcggacac 20
<210> 117
   <211> 21
   <212> DNA
   <213> Artificial Sequence
<220>
   <223> 23s reverse primer
<220>
   <221> modified_base
   <222> (21)..(21)
   <223> t-butylbenzyldA
<400> 117
   cacgtccttc atcgcctttt a 21
<210> 118
   <211> 22
   <212> DNA
   <213> Artificial Sequence
<220>
   <223> 23s reverse primer
<220>
   <221> modified_base
   <222> (22)..(22)
   <223> t-butylbenzyldC
<400> 118
   gatctcaggt ttttaccacc tc 22
<210> 119
   <211> 17
   <212> DNA
   <213> Artificial Sequence
<220>
   <223> 23s reverse primer
<220>
   <221> modified_base
   <222> (17)..(17)
   <223> t-butylbenzyldA
<400> 119
   attgctccat tcggaca 17
<210> 120
   <211> 18
   <212> DNA
   <213> Artificial Sequence
<220>
   <223> 23s reverse primer
<220>
   <221> modified_base
   <222> (18)..(18)
   <223> t-butylbenzyldC
<400> 120
   attgctccat tcggacac 18
<210> 121
   <211> 18
   <212> DNA
   <213> Artificial Sequence
<220>
   <223> 23s reverse primer
<220>
   <221> modified_base
   <222> (18)..(18)
   <223> t-butylbenzyldC
<400> 121
   attgctccat tcggacac 18
<210> 122
   <211> 19
   <212> DNA
   <213> Artificial Sequence
<220>
   <223> 23s reverse primer
<220>
   <221> modified_base
   <222> (19)..(19)
   <223> t-butylbenzyldA
<400> 122
   cgtccttcat cgcctttta 19
<210> 123
   <211> 39
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 123
   ggtcagtttg tatccagttc tgaaagaatg tttttgaac 39
<210> 124
   <211> 39
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6).. (7)
   <223> BHQ-2
<400> 124
   gttcaaaaac attctttcag aactggatac aaactgacc 39
<210> 125
   <211> 39
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 125
   gaatgttttt gaacagttct ttcaaaactg aaaacgaca 39
<210> 126
   <211> 40
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 126
   tcagtttgta tccagttctg aaagaatgtt tttgaaccag 40
<210> 127
   <211> 40
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 127
   tgttcaaaaa cattctttca gaactggata caaactgacc 40
<210> 128
   <211> 40
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 128
   agaatgtttt tgaacagttc tttcaaaact gaaaacgaca 40
<210> 129
   <211> 30
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 129
   ctaaaaggcg atgaaggacg tgttaacctg 30
<210> 130
   <211> 30
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 130
   ggtcagtttg tatccagttc tgaaagaatg 30
<210> 131
   <211> 31
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 131
   gttcaaaaac attctttcag aactggatac a 31
<210> 132
   <211> 31
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 132
   gaatgttttt gaacagttct ttcaaaactg a 31
<210> 133
   <211> 27
   <212> DNA
   <213> Artificial Sequence
<220>
   <223> 23s probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 133
   ctaaaaggcg atgaaggacg tgttaac 27
<210> 134
   <211> 27
   <212> DNA
   <213> Artificial Sequence
<220>
   <223> mgpB forward primer
<220>
   <221> modified_base
   <222> (27)..(27)
   <223> t-butylbenzyldC
<400> 134
   gacttgaaac aataacaact tctcttc 27
<210> 135
   <211> 28
   <212> DNA
   <213> Artificial Sequence
<220>
   <223> mgpB forward primer
<220>
   <221> modified_base
   <222> (28)..(28)
   <223> t-butylbenzyldA
<400> 135
   gacttgaaac aataacaact tctcttca 28
<210> 136
   <211> 28
   <212> DNA
   <213> Artificial Sequence
<220>
   <223> mgpB forward primer
<220>
   <221> modified_base
   <222> (28)..(28)
   <223> t-butylbenzyldA
<400> 136
   aacaataaca acttctcttc actaaaga 28
<210> 137
   <211> 29
   <212> DNA
   <213> Artificial Sequence
<220>
   <223> mgpB forward primer
<220>
   <221> modified_base
   <222> (29)..(29)
   <223> t-butylbenzyldA
<400> 137
   caataacaac ttctcttcac taaagatta 29
<210> 138
   <211> 26
   <212> DNA
   <213> Artificial Sequence
<220>
   <223> mgpB forward primer
<220>
   <221> modified_base
   <222> (26)..(26)
   <223> t-butylbenzyldA
<400> 138
   accccttgga cttgaaacaa taacaa 26
<210> 139
   <211> 22
   <212> DNA
   <213> Artificial Sequence
<220>
   <223> mgpB forward primer
<220>
   <221> modified_base
   <222> (22)..(22)
   <223> t-butylbenzyldA
<400> 139
   agagaaccca ggatcatttg ga 22
<210> 140
   <211> 20
   <212> DNA
   <213> Artificial Sequence
<220>
   <223> mgpB forward primer
<220>
   <221> modified_base
   <222> (20)..(20)
   <223> t-butylbenzyldA
<400> 140
   ctggagagaa cccaggatca 20
<210> 141
   <211> 26
   <212> DNA
   <213> Artificial Sequence
<220>
   <223> mgpB reverse primer
<220>
   <221> modified_base
   <222> (26).. (26)
   <223> t-butylbenzyldA
<400> 141
   gttgttatca taccttctga ttgcaa 26
<210> 142
   <211> 25
   <212> DNA
   <213> Artificial Sequence
<220>
   <223> mgpB reverse primer
<220>
   <221> modified_base
   <222> (25)..(25)
   <223> t-butylbenzyldA
<400> 142
   ctaccgttgt tatcatacct tctga 25
<210> 143
   <211> 28
   <212> DNA
   <213> Artificial Sequence
<220>
   <223> mgpB reverse primer
<220>
   <221> modified_base
   <222> (28)..(28)
   <223> t-butylbenzyldA
<400> 143
   catataaagc tctaccgttg ttatcata 28
<210> 144
   <211> 28
   <212> DNA
   <213> Artificial Sequence
<220>
   <223> mgpB reverse primer
<220>
   <221> modified_base
   <222> (28)..(28)
   <223> t-butylbenzyldA
<400> 144
   aatatcatat aaagctctac cgttgtta 28
<210> 145
   <211> 32
   <212> DNA
   <213> Artificial Sequence
<220>
   <223> mgpB reverse primer
<220>
   <221> modified_base
   <222> (32)..(32)
   <223> t-butylbenzyldA
<400> 145
   ttttccattt ttgctaagtt aatatcatat aa 32
<210> 146
   <211> 26
   <212> DNA
   <213> Artificial Sequence
<220>
   <223> mgpB reverse primer
<220>
   <221> modified_base
   <222> (26)..(26)
   <223> t-butylbenzyldA
<400> 146
   ggggttttcc atttttgcta agttaa 26
<210> 147
   <211> 36
   <212> DNA
   <213> Artificial Sequence
<220>
   <223> mgpB probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 147
   ggagagaacc caggatcatt tggattagta agaagc 36
<210> 148
   <211> 41
   <212> DNA
   <213> Artificial Sequence
<220>
   <223> mgpB probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 148
   aagattactg gagagaaccc aggatcattt ggattagtaa g 41
<210> 149
   <211> 37
   <212> DNA
   <213> Artificial Sequence
<220>
   <223> mgpB probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 149
   ctggagagaa cccaggatca tttggattag taagaag 37
<210> 150
   <211> 37
   <212> DNA
   <213> Artificial Sequence
<220>
   <223> mgpB probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 150
   cagcaaaact ttgcaatcag aaggtatgat aacaacg 37
<210> 151
   <211> 37
   <212> DNA
   <213> Artificial Sequence
<220>
   <223> mgpB probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 151
   cgttgttatc ataccttctg attgcaaagt tttgctg 37
<210> 152
   <211> 20
   <212> DNA
   <213> Artificial Sequence
<220>
   <223> MgPar forward primer
<220>
   <221> modified_base
   <222> (20)..(20)
   <223> t-butylbenzyldA
<400> 152
   tttctcccct gaatcggcaa 20
<210> 153
   <211> 20
   <212> DNA
   <213> Artificial Sequence
<220>
   <223> MgPar forward primer
<220>
   <221> modified_base
   <222> (20)..(20)
   <223> t-butylbenzyldA
<400> 153
   caactccccc tccccttcaa 20
<210> 154
   <211> 20
   <212> DNA
   <213> Artificial Sequence
<220>
   <223> MgPar forward primer
<220>
   <221> modified_base
   <222> (20)..(20)
   <223> t-butylbenzyldC
<400> 154
   tccccctccc cttcaacttc 20
<210> 155
   <211> 28
   <212> DNA
   <213> Artificial Sequence
<220>
   <223> MgPar forward primer
<220>
   <221> modified_base
   <222> (28)..(28)
   <223> t-butylbenzyldC
<400> 155
   atcccaattc agatgataat aaagtcac 28
<210> 156
   <211> 27
   <212> DNA
   <213> Artificial Sequence
<220>
   <223> MgPar forward primer
<220>
   <221> modified_base
   <222> (27)..(27)
   <223> t-butylbenzyldA
<400> 156
   atcccaattc agatgataat aaagtca 27
<210> 157
   <211> 21
   <212> DNA
   <213> Artificial Sequence
<220>
   <223> MgPar forward primer
<220>
   <221> modified_base
   <222> (21)..(21)
   <223> t-butylbenzyldA
<400> 157
   tcccaccagt gactggatca a 21
<210> 158
   <211> 20
   <212> DNA
   <213> Artificial Sequence
<220>
   <223> MgPar forward primer
<220>
   <221> modified_base
   <222> (20)..(20)
   <223> t-butylbenzyldC
<400> 158
   caactcccac actgcttccc 20
<210> 159
   <211> 23
   <212> DNA
   <213> Artificial Sequence
<220>
   <223> MgPar forward primer
<220>
   <221> modified_base
   <222> (23)..(23)
   <223> t-butylbenzyldC
<400> 159
   tccaactccc acactgcttc ccc 23
<210> 160
   <211> 21
   <212> DNA
   <213> Artificial Sequence
<220>
   <223> MgPar forward primer
<220>
   <221> modified_base
   <222> (21)..(21)
   <223> t-butylbenzyldC
<400> 160
   tccaactccc acactgcttc c 21
<210> 161
   <211> 19
   <212> DNA
   <213> Artificial Sequence
<220>
   <223> MgPar forward primer
<220>
   <221> modified_base
   <222> (19)..(19)
   <223> t-butylbenzyldC
<400> 161
   caactcccac actgcttcc 19
<210> 162
   <211> 26
   <212> DNA
   <213> Artificial Sequence
<220>
   <223> MgPar reverse primer
<220>
   <221> modified_base
   <222> (26)..(26)
   <223> t-butylbenzyldA
<400> 162
   ggtgaaaagt taggtataaa caccca 26
<210> 163
   <211> 20
   <212> DNA
   <213> Artificial Sequence
<220>
   <223> MgPar reverse primer
<220>
   <221> modified_base
   <222> (20)..(20)
   <223> t-butylbenzyldA
<400> 163
   ctgctcctgt tcagatgtca 20
<210> 164
   <211> 24
   <212> DNA
   <213> Artificial Sequence
<220>
   <223> MgPar reverse primer
<220>
   <221> modified_base
   <222> (24)..(24)
   <223> t-butylbenzyldA
<400> 164
   tgctcactat ccttgttaaa ttga 24
<210> 165
   <211> 22
   <212> DNA
   <213> Artificial Sequence
<220>
   <223> MgPar reverse primer
<220>
   <221> modified_base
   <222> (22)..(22)
   <223> t-butylbenzyldA
<400> 165
   cacctcccca aacccaggta aa 22
<210> 166
   <211> 21
   <212> DNA
   <213> Artificial Sequence
<220>
   <223> MgPar reverse primer
<220>
   <221> modified_base
   <222> (21)..(21)
   <223> t-butylbenzyldA
<400> 166
   acctccccaa acccaggtaa a 21
<210> 167
   <211> 20
   <212> DNA
   <213> Artificial Sequence
<220>
   <223> MgPar reverse primer
<220>
   <221> modified_base
   <222> (20)..(20)
   <223> t-butylbenzyldC
<400> 167
   cctgctcccg ttcagatgtc 20
<210> 168
   <211> 20
   <212> DNA
   <213> Artificial Sequence
<220>
   <223> MgPar reverse primer
<220>
   <221> modified_base
   <222> (13)..(13)
   <223> 9-(aminoethoxy)-phenoxazine-2'-dC
<220>
   <221> modified_base
   <222> (20)..(20)
   <223> t-butylbenzyldC
<400> 168
   cctgctcccg ttcaaatgtc 20
<210> 169
   <211> 20
   <212> DNA
   <213> Artificial Sequence
<220>
   <223> MgPar reverse primer
<220>
   <221> modified_base
   <222> (20)..(20)
   <223> t-butylbenzyldC
<400> 169
   cctgctcccg ttcaratgtc 20
<210> 170
   <211> 20
   <212> DNA
   <213> Artificial Sequence
<220>
   <223> MgPar reverse primer
<220>
   <221> modified_base
   <222> (20)..(20)
   <223> t-butylbenzyldC
<400> 170
   cctgctcccg ttcaaatgtc 20
<210> 171
   <211> 20
   <212> DNA
   <213> Artificial Sequence
<220>
   <223> MgPar reverse primer
<220>
   <221> modified_base
   <222> (20)..(20)
   <223> t-butylbenzyldC
<400> 171
   cctgctcccg ttcagatgtc 20
<210> 172
   <211> 40
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 172
   ctccccactt tttctaacat caatgttggg gttaaatcaa 40
<210> 173
   <211> 40
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 173
   ttgatttaac cccaacattg atgttagaaa aagtggggag 40
<210> 174
   <211> 34
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 174
   cgcaatcaac tgttgctcag aagcttacta ggaa 34
<210> 175
   <211> 35
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 175
   agttcctagt aagcttctga gcaacagttg attgc 35
<210> 176
   <211> 38
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<400> 176
   gatttaaccc caacattgat gttagaaaaa gtggggag 38
<210> 177
   <211> 38
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> modified_base
   <222> (3)..(5)
   <223> propynyldU
<220>
   <221> misc_feature
   <222> (6)..(7)
   <223> BHQ-2
<220>
   <221> modified_base
   <222> (16)..(17)
   <223> propynyldU
<220>
   <221> modified_base
   <222> (20) .. (20)
   <223> propynyldU
<220>
   <221> modified_base
   <222> (22) .. (23)
   <223> propynyldU
<220>
   <221> modified_base
   <222> (32) .. (32)
   <223> propynyldU
<400> 177
   gatttaaccc caacattgat gttagaaaaa gtggggag 38
<210> 178
   <211> 37
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6) .. (7)
   <223> BHQ-2
<400> 178
   atttaacccc aacattgatg ttagaaaaag tggggag 37
<210> 179
   <211> 37
   <212> DNA
   <213> Artificial Sequence
<220>
   <223> MgPar probe
<220>
   <221> misc_feature
   <223> 5` HEX
<220>
   <221> misc_feature
   <223> 3' phosphate
<220>
   <221> modified_base
   <222> (2) .. (4)
   <223> propynyldU
<220>
   <221> misc_feature
   <222> (6) .. (7)
   <223> BHQ-2
<220>
   <221> modified_base
   <222> (15)..(16)
   <223> propynyldU
<220>
   <221> modified_base
   <222> (19) .. (19)
   <223> propynyldU
<220>
   <221> modified_base
   <222> (21) .. (22)
   <223> propynyldU
<220>
   <221> modified_base
   <222> (31)..(31)
   <223> propynyldU
<400> 179
   atttaacccc aacattgatg ttagaaaaag tggggag 37
<210> 180
   <211> 38
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6) .. (7)
   <223> BHQ-2
<400> 180
   gatttaaccc caacattgat gttagaaaaa gtggtgag 38
<210> 181
   <211> 38
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6) .. (7)
   <223> BHQ-2
<220>
   <221> modified_base
   <222> (16)..(17)
   <223> propynyldU
<220>
   <221> modified_base
   <222> (20) .. (20)
   <223> propynyldU
<220>
   <221> modified_base
   <222> (22) .. (23)
   <223> propynyldU
<220>
   <221> modified_base
   <222> (32) .. (32)
   <223> propynyldU
<400> 181
   gatttaaccc caacattgat gttagaaaaa gtggtgag 38
<210> 182
   <211> 37
   <212> DNA
   <213> Artificial Sequence
<220>
   <223> MgPar probe
<220>
   <221> misc_feature
   <223> 5` HEX
<220>
   <221> misc_feature
   <223> 3' phosphate
<220>
   <221> misc_feature
   <222> (6) .. (7)
   <223> BHQ-2
<400> 182
   atttaacccc aacattgatg ttagaaaaag tggtgag 37
<210> 183
   <211> 37
   <212> DNA
   <213> Artificial Sequence
<220>
   <223> MgPar probe
<220>
   <221> misc_feature
   <223> 5` HEX
<220>
   <221> misc_feature
   <223> 3' phosphate
<220>
   <221> misc_feature
   <222> (6) .. (7)
   <223> BHQ-2
<220>
   <221> modified_base
   <222> (15) .. (16)
   <223> propynyldU
<220>
   <221> modified_base
   <222> (19) .. (19)
   <223> propynyldU
<220>
   <221> modified_base
   <222> (21) .. (22)
   <223> propynyldU
<220>
   <221> modified_base
   <222> (31)..(31)
   <223> propynyldU
<400> 183
   atttaacccc aacattgatg ttagaaaaag tggtgag 37
<210> 184
   <211> 37
   <212> DNA
   <213> Artificial Sequence
<220>
   <223> MgPar probe
<220>
   <221> misc_feature
   <223> 5` HEX
<220>
   <221> misc_feature
   <223> 3' phosphate
<220>
   <221> misc_feature
   <222> (6) .. (7)
   <223> BHQ-2
<220>
   <221> modified_base
   <222> (32) .. (32)
   <223> 7'deazadG
<400> 184
   atttaacccc aacattgatg ttagaaaaag tggggag 37
<210> 185
   <211> 37
   <212> DNA
   <213> Artificial Sequence
<220>
   <223> MgPar probe
<220>
   <221> misc_feature
   <223> 5` HEX
<220>
   <221> misc_feature
   <223> 3' phosphate
<220>
   <221> misc_feature
   <222> (6) .. (7)
   <223> BHQ-2
<220>
   <221> modified_base
   <222> (33) .. (33)
   <223> 7'deazadG
<400> 185
   atttaacccc aacattgatg ttagaaaaag tggggag 37
<210> 186
   <211> 37
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6) .. (7)
   <223> BHQ-2
<220>
   <221> modified_base
   <222> (34) .. (34)
   <223> 7'deazadG
<400> 186
   atttaacccc aacattgatg ttagaaaaag tggggag 37
<210> 187
   <211> 37
   <212> DNA
   <213> Artificial Sequence
<220>
   <223> MgPar probe
<220>
   <221> misc_feature
   <223> 5` HEX
<220>
   <221> misc_feature
   <223> 3' phosphate
<220>
   <221> misc_feature
   <222> (6) .. (7)
   <223> BHQ-2
<220>
   <221> modified_base
   <222> (35)..(35)
   <223> 7'deazadG
<400> 187
   atttaacccc aacattgatg ttagaaaaag tggggag 37
<210> 188
   <211> 37
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> modified_base
   <222> (2)..(4)
   <223> propynyldU
<220>
   <221> misc_feature
   <222> (6) .. (7)
   <223> BHQ-2
<220>
   <221> modified_base
   <222> (15) .. (16)
   <223> propynyldU
<220>
   <221> modified_base
   <222> (19) .. (19)
   <223> propynyldU
<220>
   <221> modified_base
   <222> (21) .. (22)
   <223> propynyldU
<220>
   <221> modified_base
   <222> (31)..(31)
   <223> propynyldU
<220>
   <221> modified_base
   <222> (32) .. (32)
   <223> 7'deazadG
<400> 188
   atttaacccc aacattgatg ttagaaaaag tggggag 37
<210> 189
   <211> 37
   <212> DNA
   <213> Artificial Sequence
<220>
   <223> MgPar probe
<220>
   <221> misc_feature
   <223> 5` HEX
<220>
   <221> misc_feature
   <223> 3' phosphate
<220>
   <221> modified_base
   <222> (2)..(4)
   <223> propynyldU
<220>
   <221> misc_feature
   <222> (6) .. (7)
   <223> BHQ-2
<220>
   <221> modified_base
   <222> (15) .. (16)
   <223> propynyldU
<220>
   <221> modified_base
   <222> (19) .. (19)
   <223> propynyldU
<220>
   <221> modified_base
   <222> (21) .. (22)
   <223> propynyldU
<220>
   <221> modified_base
   <222> (31)..(31)
   <223> propynyldU
<220>
   <221> modified_base
   <222> (33) .. (33)
   <223> 7'deazadG
<400> 189
   atttaacccc aacattgatg ttagaaaaag tggggag 37
<210> 190
   <211> 37
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> modified_base
   <222> (2)..(4)
   <223> propynyldU
<220>
   <221> misc_feature
   <222> (6) .. (7)
   <223> BHQ-2
<220>
   <221> modified_base
   <222> (15) .. (16)
   <223> propynyldU
<220>
   <221> modified_base
   <222> (19) .. (19)
   <223> propynyldU
<220>
   <221> modified_base
   <222> (21) .. (22)
   <223> propynyldU
<220>
   <221> modified_base
   <222> (31)..(31)
   <223> propynyldU
<220>
   <221> modified_base
   <222> (34)..(34)
   <223> 7'deazadG
<400> 190
   atttaacccc aacattgatg ttagaaaaag tggggag 37
<210> 191
   <211> 34
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6)..(7)
   <223> BHQ-2
<220>
   <221> modified_base
   <222> (33)..(33)
   <223> 7'deazadG
<400> 191
   atttaacccc aacattgatg ttagaaaaag tggg 34
<210> 192
   <211> 34
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> misc_feature
   <222> (6) .. (7)
   <223> BHQ-2
<400> 192
   atttaacccc aacattgatg ttagaaaaag tggg 34
<210> 193
   <211> 34
   <212> DNA
   <213> Artificial Sequence
<220>
   <223> MgPar probe
<220>
   <221> misc_feature
   <223> 5' HEX
<220>
   <221> misc_feature
   <223> 3' phosphate
<220>
   <221> modified_base
   <222> (2)..(4)
   <223> propynyldU
<220>
   <221> misc_feature
   <222> (6) .. (7)
   <223> BHQ-2
<220>
   <221> modified_base
   <222> (15) .. (16)
   <223> propynyldU
<220>
   <221> modified_base
   <222> (19) .. (19)
   <223> propynyldU
<220>
   <221> modified_base
   <222> (21) .. (22)
   <223> propynyldU
<400> 193
   atttaacccc aacattgatg ttagaaaaag tggg 34
<210> 194
   <211> 34
   <212> DNA
   <213> Artificial Sequence
<220>
   <223> MgPar probe
<220>
   <221> misc_feature
   <223> 5` HEX
<220>
   <221> misc_feature
   <223> 3' phosphate
<220>
   <221> modified_base
   <222> (2)..(4)
   <223> propynyldU
<220>
   <221> misc_feature
   <222> (6) .. (7)
   <223> BHQ-2
<220>
   <221> modified_base
   <222> (8)..(8)
   <223> propynyldC
<220>
   <221> modified_base
   <222> (10) .. (10)
   <223> propynyldC
<220>
   <221> modified_base
   <222> (13) .. (13)
   <223> propynyldC
<220>
   <221> modified_base
   <222> (15)..(16)
   <223> propynyldU
<220>
   <221> modified_base
   <222> (19) .. (19)
   <223> propynyldU
<220>
   <221> modified_base
   <222> (21).. (22)
   <223> propynyldU
<400> 194
   atttaacccc aacattgatg ttagaaaaag tggg 34